# EUROPÄISCHE PATENTSCHRIFT

(11) **EP 2 794 467 B1**
(45) Veröffentlichungstag und Bekanntmachung des Hinweises auf die Patenterteilung: **23.11.2016**
(21) Anmeldenummer: 12809618.7
(22) Anmeldetag: 20.12.2012
(51) Int. Cl.: C01B 3/24, C10J 3/00, C10J 3/72, C01B 31/02, C01B 31/18, C10K 3/06, C10G 2/00

(54) **VERFAHREN UND ANLAGE ZUR ERZEUGUNG VON SYNTHESEGAS**
PROCESS AND SYSTEM FOR GENERATING SYNTHESIS GAS
PROCÉDÉ ET INSTALLATION DE PRODUCTION DE GAZ DE SYNTHÈSE

(30) Priorität: 20.12.2011 DE 102011122562; 04.05.2012 DE 102012008933; 29.05.2012 DE 102012010542
(43) Veröffentlichungstag der Anmeldung: 29.10.2014
(73) Patentinhaber: CCP Technology GmbH, 81671 München (DE)
(72) Erfinder: KÜHL, Olaf, 17489 Greifswald (DE)
(74) Vertreter: Klang, Alexander H.
(86) Internationale Anmeldenummer: PCT/EP2012/005310
(87) Internationale Veröffentlichungsnummer: WO 2013/091879

(56) Entgegenhaltungen:
- EP-A1- 1 270 508
- EP-A2- 0 219 163
- WO-A1-00/06671
- DE-A1-102007 005 965
- GB-A- 365 912
- GB-A- 2 265 380
- GB-A- 2 265 382
- US-A1- 2003 024 806

## Beschreibung

Die vorliegende Erfindung bezieht sich auf ein Verfahren und eine Anlage zur Erzeugung von Synthesegas aus Kohlenwasserstoffen und Wasser.

Wesentliche Teile der Weltwirtschaft basieren auf Erdöl als Rohstoff- oder Energiequelle. So werden z.B. Otto- und Dieselkraftstoffe für den Individual-und Güterverkehr, schweres Heizöl für den Schiffsverkehr und als Brennstoff für Stromkraftwerke sowie leichtes Heizöl für die Beheizung von Einfamilienhäusern aus Erdöl gewonnen. Aber auch viele Rohstoffe der chemischen Industrie stammen direkt oder indirekt aus dem Erdöl. Es werden derzeit erhebliche Anstrengungen unternommen, Erdölprodukte durch andere Rohstoffe oder alternative Verfahren zu ersetzen. Auf dem Energiesektor werden beispielsweise Erdgas und erneuerbare Energien anstelle von Erdöl zum Betrieb von Stromkraftwerken eingesetzt. Im Straßenverkehr werden vor allem Elektroantrieb, Erdgasmotoren und Wasserstoffantrieb erprobt, die sich jedoch seit Jahren nicht am Markt durchzusetzen vermögen.

Es gab Versuche, Erdölprodukte in großtechnischen Verfahren aus Erdgas oder Kohle herzustellen. Beispielsweise sind Verfahren zur Umwandlung von Erdgas zu Flüssigtreibstoffen bekannt (sogenannte Gas-to-Liquid-Verfahren bzw. GtL-Verfahren), diese sind aber im Allgemeinen mit signifikanten CO₂-Emissionen oder hohen Kosten verbunden. Zudem vermögen sie es meist nicht Wasserstoff unabhängig von CO oder CO₂ bereitzustellen. Diese Versuche bleiben daher aus ökonomischen und Umweltgründen auf wenige Einzelanwendungen begrenzt.

Synthesegas, oder abgekürzt Syngas, ist eine Gasmischung, aus Kohlenmonoxid und Wasserstoff, die außerdem Kohlendioxid aufweisen kann. Das Synthesegas wird beispielsweise durch Vergasung von Kohlenstoff enthaltendem Brennstoff zu einem gasförmigen Produkt, dem Synthesegas, erzeugt, welches einen gewissen Heizwert hat. Das Synthesegas hat etwa 50% der Energiedichte von natürlichem Erdgas. Das Synthesegas kann verbrannt und somit als Brennstoffquelle verwendet werden. Das Synthesegas kann weiterhin als Zwischenprodukt zur Erzeugung von anderen chemischen Erzeugnissen verwendet werden. Synthesegas kann beispielsweise durch Vergasung von Kohle oder Abfällen erzeugt werden. Bei der Erzeugung von Synthesegas wird beispielsweise Kohlenstoff mit Wasser oder ein Kohlenwasserstoff mit Sauerstoff zur Reaktion gebracht. Es gibt kommerziell verfügbare Technologien, um Synthesegas weiterzuverarbeiten, um schließlich Industriegase, Dünger, Chemikalien und andere chemische Produkte zu erzeugen. Bei den meisten bekannten Technologien (z.B. Water-Shift-Reaktion) zur Erzeugung und Umwandlung von Synthesegas besteht jedoch das Problem, dass bei der Synthese der benötigten Menge an Wasserstoff eine größere Menge überschüssiges CO₂ erzeugt wird, welches wiederum als klimaschädliches Gas in die Umwelt gelangt. Bei einer anderen bekannten Technik zur Herstellung von Synthesegas, der partiellen Oxidation von Methan nach der Gleichung 2 CH₄ + O₂ → 2 CO + 4 H₂, kann ein maximales Verhältnis von H₂:CO von 2,0 erreicht werden. Der Nachteil ist jedoch die Verwendung von reinem Sauerstoff, der energieaufwändig hergestellt werden muss.

EP 0 219 163 A2 offenbart ein Verfahren zum Erzeugen von Synthesegas, wobei in einem ersten Reaktorraum Kohlenwasserstoffe zu Kohlenstoff und Wasserstoff aufgespaltet werden und der Kohlenstoff zur Reaktion mit Wasserdampf in einem zweiten Reaktionsraum überführt wird.

In WO 00/06671 A1 ist ein Verfahren zum Erzeugen von Synthesegas beschrieben, bei dem in einem ersten Reaktor aus Biomasse unter Luftzufuhr elementarer Kohlenstoff und Abfallgase wie Wasser und CO₂ erzeugt werden, wobei aus dem Kohlenstoff in einem zweiten Reaktor unter Zufuhr von Wasserdampf Synthesegas erzeugt wird.

Es ist daher eine erste Aufgabe der Erfindung, ein Kohlenwasserstoff enthaltenden Fluid in Synthesegas mit einem variablen Wasserstoffgehalt umzuwandeln ohne nennenswerte Mengen von CO₂ zu erzeugen.
Die Aufgabe der Erfindung wird gelöst durch die Verfahren nach den Ansprüchen 1 und 2 sowie durch die Vorrichtung nach den Ansprüchen 9 und 10. Weitere Ausgestaltungen ergeben sich aus den Unteransprüchen.

Insbesondere weist ein Verfahren zum Erzeugen von Synthesegas das Aufspalten eines Kohlenwasserstoff enthaltenden Fluids zu Kohlenstoff und Wasserstoff durch einen Energieeintrag auf, der wenigstens teilweise durch Wärme geleistet wird, wobei der Kohlenstoff und der Wasserstoff nach der Aufspaltung eine Temperatur von wenigstens 200°C besitzt. Ein Teil des Kohlenstoffs, der aus der Aufspaltung gewonnen wurde, wird dann bei einer Temperatur von 800 bis 1700°C in Kontakt mit Wasser gebracht, wobei sich der durch die Aufspaltung gewonnene Kohlenstoff beim In-Kontakt-Bringen mit dem Wasser um höchstens 50% in °C hinsichtlich seiner Temperatur nach der Aufspaltung abgekühlt hat. Hierbei wird wenigstens ein Teil des Wassers und des durch die Aufspaltung gewonnenen Kohlenstoffes in ein Synthesegas umgewandelt. Durch dieses Verfahren kann ein Kohlenwasserstoff enthaltendes Fluid in ein Synthesegas mit einem variablen Wasserstoffgehalt umgewandelt werden ohne nennenswerte Mengen von CO₂ zu erzeugen. In vorteilhafter Weise wird dabei wenigstens ein Teil einer für die Bereitstellung von Kohlenstoff (durch Aufspaltung eines Kohlenwasserstoffs) erforderlichen Energie in Form von Wärme für die Umwandlung eingesetzt. Als Nebenprodukte können zusätzlich Wasserstoff und Kohlenstoff in verschiedenen Formen gewonnen werden. Gemäß der Erfindung kann der durch die Aufspaltung gewonnene Wasserstoff gemeinsam mit dem Wasser in Kontakt gebracht werden, da Wasserstoff die Umwandlung nicht beeinträchtigt. Er kann als zusätzlicher Wärmeträger dienen, was besonders vorteilhaft ist, wenn der Kohlenstoff und der Wasserstoff eine Temperatur von 1000°C (einer bevorzugten Umwandlungstemperatur) oder höher besitzen. In diesem Fall liegt nach der Umwandlung kein Wassergas sondern ein Synthesegas mit einem anderen Mischungsverhältnis vor. Alternativ kann der durch die Aufspaltung gewonnene Kohlenstoff vor dem In-Kontakt-Bringen mit dem Wasser von dem durch die Aufspaltung gewonnenen Wasserstoff wenigstens teilweise getrennt und dem durch die Umwandlung entstehenden Synthesegas zumindest teilweise beigemischt werden.

Dies gilt insbesondere, wenn die Aufspaltung bei einer Temperatur über 1000°C erfolgt und der Kohlenstoff mit einer Temperatur von wenigstens 1000°C, insbesondere bei einer Temperatur zwischen 1000°C und 1200°C mit dem Wasser in Kontakt gebracht wird, da in diesem Fall keine oder nur eine geringere zusätzliche Wärmemenge für die Umwandlung bereitgestellt werden muß. Bevorzugt wird die erforderliche Wärme zum Erreichen der Temperatur von 800 bis 1700°C (insbesondere von 1000°C bis 1200°C) für die Umwandlung im Wesentlichen vollständig durch die Wärme, die für die Aufspaltung des Kohlenwasserstoff enthaltenden Fluids eingesetzt wird, bereitgestellt. Im Wesentlichen bedeutet hier, dass wenigstens 80%, insbesondere wenigstens 90% der erforderlichen Wärme aus dem Aufspaltungsprozeß stammen.

Zum Erhöhen der Energieeffizienz des Verfahrens kann wenigstens ein Teil der Wärme wenigstens eines Teils des durch die Aufspaltung gewonnenen Kohlenstoffs und/oder Wasserstoffes zum Erwärmen des Wassers vor dem In-Kontakt-Bringen mit dem Kohlenstoff und/oder zum Erwärmen eines Prozeßraums, in dem das Wasser mit dem Kohlenstoff in Kontakt gebracht wird, verwendet werden. In diesem Sinne sei auch bemerkt, dass das Synthesegas nach der Umwandlung eine Temperatur von 800 bis 1700°C aufweist und wenigstens ein Teil seiner Wärme zum Vorwärmen des Wassers vor dem In-Kontakt-Bringen mit dem Kohlenstoff verwendet werden kann. Auch ist es möglich, dass wenigstens ein Teil der Wärme wenigstens eines Teils des durch die Aufspaltung gewonnenen Kohlenstoffs und/oder Wasserstoffes und/oder des Synthesegases nach der Umwandlung zur Erzeugung von elektrischem Strom verwendet wird, der insbesondere als Energieträger für den Energieeintrag für das Aufspalten des Kohlenwasserstoff enthaltenden Fluids bereitgestellt werden kann.

Bevorzugt erfolgt der Energieeintrag in den Kohlenwasserstoff zu seiner Aufspaltung primär über ein Plasma. Dies ist eine besonders direkte und somit effiziente Form des Energieeintrags. Bevorzugt wird die Aufspaltung in einem Kvaerner-Reaktor durchgeführt, der eine kontinuierliche Aufspaltung eines Stromes an Kohlenwasserstoffen ermöglicht.

Bei dem Verfahren zum Erzeugen eines Synthesegases kann dem Synthesegas nach der Umwandlung zusätzlicher Wasserstoff und/oder Kohlenmonoxid und/oder ein weiteres Synthesegas beigemischt werden, um eine gewünschte Zusammensetzung zu erreichen. Für den Fall des gemeinsamen In-Kontakt-Bringen von Kohlenstoff und Wasserstoff mit Wasser kann es besonders zweckmäßig sein dem Synthesegas zusätzliches Kohlenmonoxid beizumischen, um das CO:H₂ Verhältnis zu reduzieren. Beim In-Kontakt-Bringen von im wesentlichen reinen Kohlenstoff mit Wasser kann es hingegen zweckmäßig sein dem Synthesegas zusätzlichen Wasserstoff beizumischen, um das CO:H₂ Verhältnis zu erhöhen. Insbesondere ist es auch möglich die Ströme zweier separat in obiger Weise erzeugter Synthesegase (einer mit, einer ohne vorheriger Trennung des Kohlenstoffs und des Wasserstoffs) zu vermischen, um ein gewünschtes Mischungsverhältnis von CO:H₂ zu erhalten.

Bevorzugt stammt der zusätzliche Wasserstoff aus einer Aufspaltung eines Kohlenwasserstoff enthaltenden Fluids zu Kohlenstoff und Wasserstoff durch einen Energieeintrag, der wenigstens teilweise durch Wärme geleistet wird. Die Aufspaltung kann somit einerseits den erforderlichen Kohlenstoff zur C-Wasser-Umwandlung und anderseits den erforderlichen Wasserstoff in einem Arbeitsgang zur Verfügung stellen. Bei einer Ausführungsform wird wenigstens ein Teil des Wasserstoffs durch Aufspalten eines Kohlenwasserstoff enthaltenden Fluids bei einer Temperatur unter 1000°C, insbesondere unter 600°C mittels eines Mikrowellenplasmas erzeugt. Dort wo zusätzlicher Wasserstoff (mehr als bei der Herstellung des für die C-Wasser-Umwandlung erforderlichen Kohlenstoffs anfällt) für die Einstellung eines Mischungsverhältnisses eines Synthesegases benötigt wird, kann dieser bevorzugt energieeffizient bei niedrigeren Temperaturen aus einem Kohlenwasserstoff enthaltenden Fluid abgespalten werden. Bevorzugt wird das Verhältnis CO zu Wasserstoff im Synthesegas auf einen Wert von 1:1 bis 1:3, insbesondere auf einen Wert von ungefähr 1:2,1 eingestellt.

Bei einem Verfahren zum Erzeugen von synthetischen, funktionalisierten und/oder nicht-funktionalisierten Kohlenwasserstoffen, wird zunächst ein Synthesegas wie oben beschrieben erzeugt und dieses mit einem geeigneten Katalysator in Kontakt gebracht, um eine Umwandlung des Synthesegases in synthetische, funktionalisierte und/oder nicht-funktionalisierte Kohlenwasserstoffe zu bewirken, wobei die Temperatur des Katalysators und oder des Synthesegases auf einen vorbestimmten Temperaturbereich gesteuert oder geregelt wird. Dabei kann das Synthesegas vorab oder auch direkt im Katalysator durch Mischen von CO mit Wasserstoff erzeugt werden.

Bei einer Ausführungsform wird die Umwandlung des Synthesegases mittels eines Fischer-Tropsch-Verfahrens, insbesondere mittels eines SMDS-Verfahrens bewirkt. Alternativ wird die Umwandlung des Synthesegases mittels eines Bergius-Pier-Verfahrens, eines Pier-Verfahrens oder einer Kombination aus einem Pier-Verfahren mit einem MtL-Verfahren bewirkt. Die Wahl des Verfahrens bestimmt im Wesentlichen die Form der synthetischen, funktionalisierten und/oder nicht-funktionalisierten Kohlenwasserstoffe. Bevorzugt ist das aufzuspaltende, ein Kohlenwasserstoff enthaltende Fluid Erdgas, Methan, Flüssiggas, Schweröl oder eine Mischung derselben.

Die Vorrichtung zum Erzeugen eines Synthesegases besitzt einen Kohlenwasserstoffkonverter zum Aufspalten eines Kohlenwasserstoff enthaltenden Fluids in Kohlenstoff und Wasserstoff, der wenigstens einen Prozessraum mit wenigstens einem Eingang für ein Kohlenwasserstoff enthaltendes Fluid und wenigstens einem Ausgang für Kohlenstoff und/oder Wasserstoff und wenigstens eine Einheit zum Einbringen von Energie in den Prozessraum, die wenigstens teilweise aus Wärme besteht, aufweist. Ferner weist die Vorrichtung einen C-Konverter zur Umwandlung von Wasser und Kohlenstoff auf, der wenigstens einen weiteren Prozessraum mit wenigstens einem Eingang für Wasser, wenigstens einem Eingang für wenigstens Kohlenstoff und wenigstens einem Ausgang aufweist, wobei der Eingang für wenigstens Kohlenstoff direkt mit dem wenigstens einem Ausgang des Kohlenwasserstoffkonverters verbunden ist. Dabei soll direkt hier umschreiben, dass im Betrieb aus dem Kohlenwasserstoffkonverter austretender Kohlenstoff auf seinem Weg zum C-Konverter um nicht mehr als 50% seiner Temperatur in °C, bevorzugt um nicht mehr als 20% abkühlt, ohne dass zusätzliche Energie zum Wärmen des Kohlenstoffs eingesetzt wird. Der Kohlenwasserstoffkonverter, sein Ausgang, der der C-Konverter und sein Eingang sind bei einer Ausführungsform so ausgebildet, dass im Kohlenwasserstoffkonverter ausgebildeter Kohlenstoff und Wasserstoff gemeinsam in den C-Konverter geleitet werden. Bei einer anderen Ausführungsform ist eine Trenneinheit zum Trennen des durch die Aufspaltung entstandenen Kohlenstoffs und des Wasserstoffs und getrennte Ausgänge für die getrennten Stoffe aus der Trenneinheit vorgesehen, wobei der Ausgang für Kohlenstoff mit dem C-Konverter verbunden ist. Bei dieser Ausführung ist ferner eine separate Zuleitung für den Wasserstoff aus der Trenneinheit in einen stromabwärts befindlichen Mischraum vorgesehen. Die Trenneinheit kann zwischen dem Ort der Aufspaltung und dem wenigstens einen Ausgang des Kohlenwasserstoffkonverters eine vorgesehen sein. Diese kann einen Teil des Kohlenwasserstoffkonverters bilden oder auch außerhalb des Kohlenwasserstoffkonverters als separate Einheit vorgesehen sein. Eine Trenneinheit zwischen dem Ausgang des Kohlenwasserstoffkonverters und dem Eingang eines C-Konverters behindert eine direkte Verbindung nicht, solange die obige Bedingung beibehalten wird.

Bevorzugt ist die wenigstens eine Einheit zum Einbringen von Energie in den Prozessraum so ausgebildet, dass sie wenigstens lokal Temperaturen über 1000°C, insbesondere über 1500°C erzeugen kann. Bei einer Ausführungsform ist die wenigstens eine Einheit zum Einbringen von Energie in den Prozessraum eine Plasmaeinheit. Insbesondere wenn die Temperatur für die Aufspaltung unter 1000°C gehalten werden soll, weist die wenigstens eine Einheit zum Einbringen von Energie in den Prozessraum bevorzugt eine Mikrowellen-Plasmaeinheit auf.

Für eine besonders einfache Ausgestaltung der Vorrichtung wird der Prozessraum des C-Konverters durch ein Auslaßrohr des Kohlenwasserstoffkonverters gebildet, das mit einer Einspeisung für Wasser verbunden ist.

Bei dem Kohlenwasserstoffkonverter handelt es sich bevorzugt um einen Kvaerner-Reaktor, der die erforderlichen Temperaturen im Dauerbetrieb für eine kontinuierliche Aufspaltung eines Kohlenwasserstoff enthaltenden Fluids vorsehen kann.

Bei einer Ausführungsform weist die Vorrichtung zum Erzeugen eines Synthesegases wenigstens einen weiteren Kohlenwasserstoffkonverter zum Aufspalten eines Kohlenwasserstoff enthaltenden Fluids in Kohlenstoff und Wasserstoff auf. Der wenigstens eine weitere Kohlenwasserstoffkonverter weist wieder wenigstens einen Prozessraum mit wenigstens einem Eingang für das Kohlenwasserstoff enthaltende Fluid, wenigstens eine Einheit zum Einbringen von Energie in den Prozessraum, die wenigstens teilweise aus Wärme besteht, und eine Trenneinheit zum Trennen des durch die Aufspaltung entstandenen Kohlenstoffs und des Wasserstoffs mit getrennten Ausgängen für Kohlenstoff und Wasserstoff auf, wobei der Ausgang für Wasserstoff mit der separaten Zuleitung für Wasserstoff verbunden ist. Dabei ist der wenigstens eine weitere Kohlenwasserstoffkonverter aus Gründen der Energieeffizienz bevorzugt des Typs, der ein Aufspaltung bei Temperaturen unter 1000°C, insbesondere unter 600°C mittels eines Mikrowellenplasmas bewirkt.

Bei der Vorrichtung zum Umwandeln eines Synthesegases in synthetische, funktionalisierte und/oder nicht-funktionalisierte Kohlenwasserstoffe, ist eine Vorrichtung zum Erzeugen eines Synthesegases des obigen Typs und ein CO-Konverter vorgesehen. Der CO-Konverter weist einen Prozessraum, in dem ein Katalysator angeordnet ist, Mittel zum Leiten des Synthesegases in Kontakt mit dem Katalysator, und eine Steuereinheit zum Steuern oder Regeln der Temperatur des Katalysators und/oder des Synthesegases auf eine vorbestimmte Temperatur auf. Dabei können Teile der Vorrichtung zum Erzeugen eines Synthesegases im CO-Konverter integriert sein, wie beispielsweise ein Mischraum für CO und zusätzlichen Wasserstoff, Kohlenstoff und/oder ein anderes Synthesegas. Bei einer Ausführungsform weist der CO-Konverter einen Fischer-Tropsch-Konverter, insbesondere einen SMDS-Konverter auf. Alternativ kann der CO-Konverter auch ein Bergius-Pier-Konverter, einen Pier-Konverter oder eine Kombination eines Pier-Konverters mit einen MtL-Konverter aufweisen. Auch ist es möglich, dass mehrere CO-Konverter desselben oder auch unterschiedlicher Typen in der Vorrichtung vorhanden sind.

Bevorzugt weist die Vorrichtung eine Steuereinheit zum Steuern oder Regeln des Drucks des Synthesegases im CO-Konverter auf.

Die Erfindung wird nachfolgend unter Bezugnahme auf bestimmte Ausführungsformen anhand der Zeichnungen näher erläutert; in den Zeichnungen zeigt:
- Fig. 1: eine schematische Darstellung einer Anlage zur Erzeugung von Synthesegas;
- Fig. 2: eine schematische Darstellung einer alternativen Anlage zur Erzeugung von Synthesegas;
- Fig. 3: eine schematische Darstellung einer Anlage zur Erzeugung von funktionalisiertem und/oder nicht-funktionalisiertem Kohlenwasserstoff;
- Fig. 4: eine schematische Darstellung einer weiteren Anlage zur Erzeugung von funktionalisiertem und/oder nicht-funktionalisiertem Kohlenwasserstoff gemäß einer weiteren Ausführungsform;
- Fig. 5: eine schematische Darstellung einer Anlage zur Erzeugung von funktionalisiertem und/oder nicht-funktionalisiertem Kohlenwasserstoff gemäß einer weiteren Ausführungsform;
- Fig. 6: eine schematische Darstellung einer Anlage zur Erzeugung von funktionalisiertem und/oder nicht-funktionalisiertem Kohlenwasserstoff gemäß einer weiteren Ausführungsform;
- Fig. 7: eine schematische Darstellung einer Anlage zur Erzeugung von Synthesegas gemäß einer weiteren Ausführungsform; und
- Fig. 8: eine schematische Darstellung einer Anlage zur Erzeugung von funktionalisiertem und/oder nicht-funktionalisiertem Kohlenwasserstoff gemäß einer weiteren Ausführungsform.

Es sei bemerkt, dass sich in der folgenden Beschreibung die Ausdrücke oben, unten, rechts und links sowie ähnliche Angaben auf die in den Figuren dargestellten Ausrichtungen bzw. Anordnungen beziehen und nur zur Beschreibung der Ausführungsbeispiele dienen. Diese Ausdrücke können bevorzugte Anordnungen zeigen, sind jedoch nicht im einschränkenden Sinne zu verstehen.

Ferner werden in den unterschiedlichen Figuren zum Teil dieselben Bezugszeichen verwendet, sofern gleiche oder ähnliche Teile bezeichnet werden.

In der folgenden Beschreibung werden Prozesse und Vorrichtungen beschrieben, die "heiße" Stoffe oder "heiße" Prozesse ausführen. Im Zusammenhang mit dieser Beschreibung soll der Ausdruck "heiß" eine Temperatur über 200°C und vorzugsweise über 300°C beschreiben.

Synthesegas ist jedes Gas, das im Wesentlichen aus Kohlenmonoxid und Wasserstoff besteht. Als Wassergas wird ein (Synthese-)Gas bezeichnet, das zu nahezu gleichen Teilen aus Kohlenmonoxid und Wasserstoff besteht (1:1). Wenn hier von Synthesegas gesprochen wird, umfaßt dieser Ausdruck also auch Wassergas als spezielle Mischung eines Synthesegases.

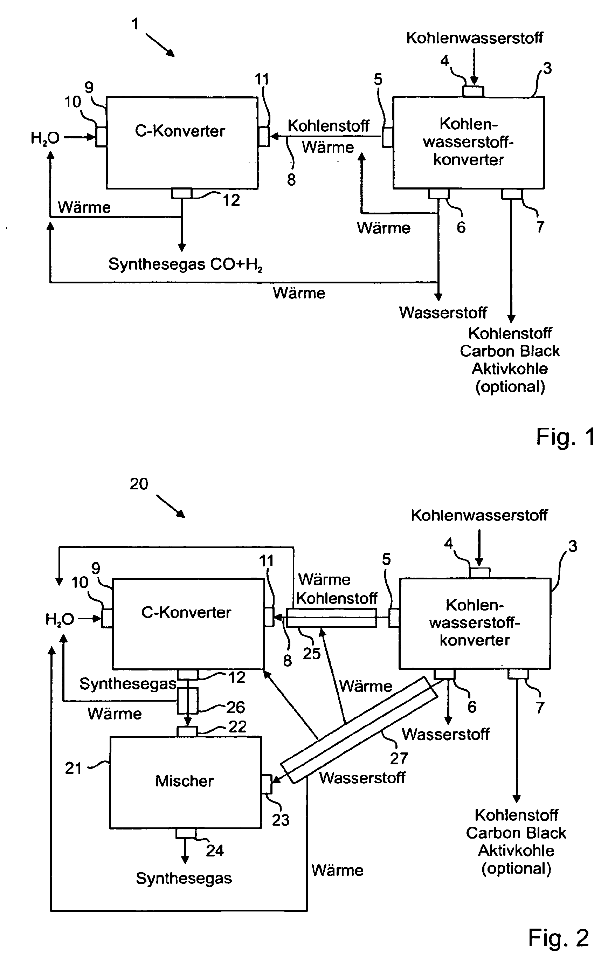

Fig. 1 stellt schematisch eine Anlage 1 zur Erzeugung von Synthesegas dar. Aus der Fig. 1 wird auch der grundlegende Verfahrensablauf zur Erzeugung von Synthesegas gemäß dieser Beschreibung deutlich.

Die Anlage 1 zur Erzeugung von Synthesegas weist einen Kohlenwasserstoffkonverter 3 auf, welcher einen Kohlenwasserstoffeingang 4 sowie einen ersten Kohlenstoffausgang 5, einen optionalen Wasserstoffausgang 6 sowie einen optionalen zweiten Kohlenstoffausgang 7 aufweist. Die Anlage 1 zur Erzeugung von Synthesegas weist weiter einen C-Konverter 9 mit einem Wasser-Eingang 10 einem Kohlenstoffeingang 11 (auch als C-Eingang bezeichnet) und einen Synthesegasausgang 12 (Synthesegas-Ausgang) auf. Der Kohlenwasserstoffkonverter 3 und der C-Konverter 9 sind derart angeordnet, dass der Kohlenstoffausgang 5 des Kohlenwasserstoffkonverters 3 über eine direkte Verbindung 8 mit dem Kohlenstoffeingang 11 des C-Konverters 9 verbunden ist, wobei der Ausgang 5 auch direkt den Kohlenstoffeingang 11 des C-Konverters 9 bilden kann. So kann Kohlenstoff aus dem Kohlenwasserstoffkonverter 3 direkt in den C-Konverter 9 transportiert werden.

Der Kohlenwasserstoffkonverter 3 ist irgendein Kohlenwasserstoffkonverter, der eingespeiste Kohlenwasserstoffe in Kohlenstoff und Wasserstoff umwandeln bzw. aufspalten kann. Der Kohlenwasserstoffkonverter 3 weist einen Prozessraum mit einem Einlaß für ein Kohlenwasserstoff enthaltendes Fluid, wenigstens eine Einheit zum Einbringen von Aufspaltungsenergie in das Fluid und wenigstens einen Auslaß auf. Die Aufspaltungsenergie wird wenigstens teilweise durch Wärme zur Verfügung gestellt, die beispielsweise durch ein Plasma erzeugt wird. Sie kann aber auch auf andere Weise zur Verfügung gestellt werden und wenn primär eine Aufspaltung über Wärme erfolgt, so sollte das Fluid auf über 1000°C insbesondere auf eine Temperatur über 1500°C aufgeheizt werden.

Bei der dargestellten Ausführungsform wird ein Kvaerner Reaktor eingesetzt, der mittels eines Plasmabogens die erforderliche Wärme zur Verfügung stellt. Es sind aber auch andere Reaktoren bekannt, die bei niedrigeren Temperaturen insbesondere unter 1000°C arbeiten und neben der Wärme zusätzliche Energie in den Kohlenwasserstoff einbringen, wie beispielsweise über ein Mikrowellenplasma. Wie Nachfolgend noch näher erläutert wird, zieht die Erfindung beide Reaktortypen (und auch solche die ohne ein Plasma arbeiten) in betracht, insbesondere auch in Kombination miteinander. Kohlenwasserstoffkonverter die bei einer Temperatur von mehr als 1000°C arbeiten werden nachfolgend als Hochtemperatur-Reaktoren bezeichnet, während solche, die bei Temperaturen unter 1000°C arbeiten, insbesondere bei einer Temperatur zwischen 200°C und 1000°C, als Niedertemperatur-Reaktoren bezeichnet werden.

In dem Reaktor werden mittels Wärme und/oder einem Plasma Wasserstoff und Kohlenstoff aus Kohlenwasserstoffen (CₙHₘ) aufgetrennt.. Die Kohlenwasserstoffe werden dabei bevorzugt in Gasform in den Reaktor eingebracht. Bei unter Normalbedingungen flüssigen Kohlenwasserstoffen können diese vor dem Einbringen in den Reaktor in Gasform gebracht werden, oder sie könnten auch in einer fein zerstäubten Form eingeleitet werden. Beide Formen werden nachfolgend als Fluide bezeichnet.

Die Auftrennung der Kohlenwasserstoffe sollte möglichst unter Ausschluß von Sauerstoff, um die unerwünschte Bildung von Kohlenstoffoxiden oder Wasser zu unterbinden. Geringe Mengen an Sauerstoff, die beispielsweise mit dem Kohlenwasserstoffen eingebracht werden, sind aber auch wiederum für den Prozeß nicht schädlich.

Bei dem oben beschriebenen Kvaerner Reaktor werden Kohlenwasserstoff enthaltende Fluide in einem Plasmabrenner bei hoher Temperatur in reinen Kohlenstoff (beispielsweise in Form von Aktivkohle, Carbon Black, Graphit oder Industrieruß) und Wasserstoff und ggf. Verunreinigungen getrennt. Die Kohlenwasserstoffe enthaltenden Fluide als Eingangsstoffe für den Kohlenwasserstoffkonverter 3 sind beispielsweise Methan, Erdgas, Biogase, Flüssiggase oder Schweröl. Es können aber auch synthetische, funktionalisierte und/oder nicht-funktionalisierte Kohlenwasserstoffe als Eingangsstoffe für den Kohlenwasserstoffkonverter 3 verwendet werden. Nach der ursprünglichen Trennung liegen die Elemente in der Regel als eine Mischung, insbesondere in Form eines Aerosols vor. Diese Mischung kann, wie nachfolgend beschrieben wird, in dieser Form einem weiteren Prozeß zugeführt werden, oder sie kann auch in einer entsprechenden, nicht dargestellten Trenneinheit in ihre Einzelelemente getrennt werden. Eine solche Trenneinheit wird im Rahmen dieser Anmeldung als Teil des Kohlenwasserstoffkonverters 3 angesehen, auch wenn sie natürlich auch als getrennte Einheit ausgeführt sein kann. Wenn keine Trenneinheit vorgesehen ist, dann ist der Kohlenstoffausgang 5 der einzige Ausgang des Kohlenwasserstoffkonverters 3, der eine Mischung (ein Aerosol) aus Kohlenstoff und Wasserstoff direkt in den C-Konverter 9 leitet. Mit einer Trenneinheit, kann über den Kohlenstoffausgang 5 wenigstens teilweise vom Wasserstoff getrennter Kohlenstoff in den C-Konverter 9 geleitet werden. Über die optionalen Ausgänge 6 und 7 können dann abgetrennter Wasserstoff und ggf. weiterer Kohlenstoff ausgeleitet werden.

Der C-Konverter 9 kann irgendein geeigneter C-Konverter sein, der Synthesegas (Syngas) aus Kohlenstoff (C) und Wasser (H₂O) erzeugen kann. In der Ausführungsform der Fig. 1 wird im C-Konverter 9 H₂O über heißen Kohlenstoff geleitet oder auch als Wasserdampf in einem Strom aus Kohlenstoff und Wasserstoff eingeleitet und hiermit vermischt, um gemäß der chemischen Gleichung C + H₂O → CO + H₂ umgewandelt zu werden. Im C-Konverter 9 laufen folgende Reaktionen ab:

C + H₂O → CO + H₂ + 131,38 kJ/mol endotherm

CO + H₂O → CO₂ + H₂ - 41,19 kJ/mol exotherm

Im Boudouard-Gleichgewicht erfolgt die Reaktion:

2 C + O₂ → 2 CO + 172,58 kJ/mol endotherm

Da alle drei Reaktionen im Gleichgewicht miteinander stehen, findet der Prozeß im C-Konverter 9 vorzugsweise bei hohen Temperaturen von 800 bis 1700°C, vorzugsweise 1000 bis 1200°C statt, da bei niedriger Temperatur die zweite Reaktion bevorzugt werden würde, wobei die für das Erreichen dieser Temperatur erforderliche Wärme primär durch das Ausgangsmaterial des Kohlenwasserstofflconverters 3 zu Verfügung gestellt wird, wie nachfolgend noch näher erläutert wird. Das Wasser (H₂O) im C-Konverter 9 ist bei diesen Bedingungen dampfförmig und kann auch schon in Dampfform eingeleitet werden. Die Zugabe von Wasser wird im Betrieb der Anlage 1 so gesteuert, dass ein Überschuss an Wasser vermieden wird, um ein starkes Abkühlen zu vermeiden. Bei einer übermäßigen Abkühlung im C-Konverter 9 würde ebenfalls die zweite Reaktion bevorzugt ablaufen.

Der C-Konverter 9 arbeitet am besten bei hohen Temperaturen von 1000 bis 1200°C, um die exotherme Water-Shift-Reaktion CO + H₂O → CO₂ + H₂ zurückzudrängen und so den Anteil an Kohlenmonoxid im Synthesegas zu optimieren. Die Reaktionen im C-Konverter 9 sind dem Fachmann bekannt und werden daher hier nicht genauer beschrieben.

Der Betrieb der Anlage 1 zur Erzeugung von Synthesegas wird nachfolgend unter Bezugnahme auf die Fig. 1 näher erläutert. Dabei wird im Nachfolgenden davon ausgegangen, dass der Kohlenwasserstoffkonverter 3 ein Hochtemperatur Reaktor des Kvaerner Typs ist. Kohlenwasserstoffe enthaltende Fluide (insbesondere in Gasform) werden über den Kohlenwasserstoffeingang 4 in den Kohlenwasserstoffkonverter 3 eingeleitet. Wenn der Kohlenwasserstoff beispielsweise Methan (CH₄) ist, so entstehen 1 mol Kohlenstoff und 2 mol Wasserstoff aus 1 mol Methan. Die Kohlenwasserstoffe werden in dem Plasma-Brenner des Kohlenwasserstoffkonverters 3 bei etwa 1600°C gemäß der folgenden Reaktionsgleichung umgewandelt, wobei die zugeführte Energie Wärme ist, die im Plasma mittels elektrischer Energie erzeugt wird:

CₙHₘ + Energie → n C + m/2 H₂

Bei entsprechender Prozeßführung ist der Kvaerner Reaktor in der Lage im kontinuierlichen Betrieb eine nahezu 100% Umwandlung des Kohlenwasserstoffs in seine Bestandteile zu erreichen.

Im Nachfolgenden wird davon ausgegangen, dass der Kohlenstoff und der Wasserstoff im Kohlenwasserstoffkonverter 3 getrennt und weitestgehend getrennt ausgeleitet werden. Es ist aber auch möglich, dass eine Trennung nicht erfolgt, und der Kohlenstoff und der Wasserstoff als Gemisch ausgeleitet und dem C-Konverter 9 zugeführt werden. Der Wasserstoff beeinträchtigt nicht den Umwandlungsprozeß im C-Konverter 9, kann aber als zusätzlicher Wärmeträger dienen. Der Kohlenstoff wird zumindest teilweise direkt über den Kohlenstoffausgang 5 in den Kohlenstoffeingang 11 des C-Konverter 9 eingeleitet. Dabei soll das "direkte" leiten vom Ausgang 5 des Kohlenwasserstoffkonverters 3 zum Kohlenstoffeingang 11 des C-Konverters 9 alle solche Varianten umfassen, bei denen keine Abkühlung von mehr als 50% bezogen auf die Temperatur (vorzugsweise von nicht mehr als 80%) der geleiteten Stoffe auftritt. Da der aus dem Kohlenwasserstoffkonverter 3 austretende Kohlenstoff eine hohe Temperatur, bevorzugt über 1000°C aufweist, kann die darin enthaltene Wärmeenergie zum Erhalten der erforderlichen Temperatur für den Umwandlungsprozesses im C-Konverter 9 verwendet werden, der beispielsweise bei einer Temperatur von ca. 1000°C arbeitet.

Die Verbindung 8 zwischen dem Kohlenwasserstoffkonverter 3 und dem C-Konverter 9 ist so ausgestaltet, dass sich der Kohlenstoff auf dem Weg vom Kohlenwasserstoffkonverter 3 zum C-Konverter 9 nicht stark abkühlt (um weniger als 50%, bevorzugt weniger als 20% bezogen auf die Temperatur). Beispielsweise kann die Verbindung 8 besonders isoliert und/oder sogar aktiv beheizt sein, wobei dem System bevorzugt - neben der Wärmezufuhr im Kohlenwasserstoffkonverter 3 - keine weitere Wärme zugeführt wird.. Der im Kohlenwasserstoffkonverter 3 erzeugte Wasserstoff enthält aufgrund der Betriebstemperatur im Kohlenwasserstoffkonverter 3 ebenfalls Wärmeenergie. Daher besteht eine Möglichkeit des Beheizens der Verbindung 8 darin, die Wärmeenergie des aus dem Wasserstoffausgang 6 herausgeleiteten Wasserstoffes, direkt oder indirekt über eine Wärmetauscheranordnung zum Beheizen der Verbindung 8 zwischen Kohlenwasserstoffkonverter 3 und C-Konverter 9 zu verwenden.

Im C-Konverter 9 wird Wasser, insbesondere in Dampfform, das über den Wasser-Eingang 10 des C-Konverters 9 eingeleitet wird, über den heißen Kohlenstoff geleitet und/oder mit diesem vermischt. Der C-Konverter 9 arbeitet am besten bei hohen Temperaturen, da es sich um eine endotherme Reaktion handelt und die Water-Shift-Reaktion, die zu ihr in Konkurrenz steht eine exotherme Reaktion ist. Die Reaktion, welche dem Fachmann bekannt ist, ist abhängig von Druck und Temperatur und wird hier nicht im Einzelnen beschrieben. Entweder die Menge des in den C-Konverter 9 eingeleiteten Wassers oder die Menge des Kohlenstoffs kann über geeignete Mittel gesteuert und/oder geregelt werden.

C + H₂O → CO + H₂; ΔH = +131,38 kJ/mol

Allerdings ist auch hier das Boudouard-Gleichgewicht der begrenzende Faktor, weswegen bei Temperaturen oberhalb von 1000°C und in Abwesenheit eines Wasserüberschusses fast ausschließlich ein Gemisch von Kohlenmonoxid und Wasserstoff vorliegt. Es ist vorteilhaft, das in den Wasser-Eingang 10 des C-Konverters 9 eingeleitete Wasser vorzuwärmen, da der C-Konverter 9 bevorzugt bei einer Temperatur > 1000°C arbeitet. Eine Vorwärmung des Wassers kann beispielsweise erreicht werden, indem die in dem heißen Wasserstoff enthaltene Wärmeenergie direkt oder indirekt über eine Wärmetauscheranordnung zum Vorwärmen des Wassers verwendet wird. Bevorzugt reicht aber auch die im Kohlenstoff enthaltene Wärme, um das Wasser auf die gewünschte Temperatur zu bringen. Nur für den Fall, dass die im Kohlenwasserstoffkonverter 3 erzeugte Wärme nicht ausreicht, um die gewünschte Umwandlungstemperatur von ungefähr 1000°C zu erreichen, kann eine optionale zusätzliche Heizeinheit zum Erwärmen des C-Konverters 9 oder der darin befindlichen Elemente vorgesehen sein. Eine solche kann auch als eine Vorheizeinheit im Bereich einer Zuleitung für das Wasser oder den Kohlenstoff eingesetzt werden. Sie kann auch nur für den Start der Anlage eingesetzt werden, um zunächst den C-Konverter 9 oder medienführende Teile der Anlage auf eine Anfangstemperatur zu bringen, damit das System schneller einen gewünschten Temperaturzustand erreicht. Die Aufheizung aller medienführenden Teile rein über die im Kohlenwasserstoffkonverter 3 erzeugte Wärme könnte anfangs zu lange dauern

Aus dem C-Konverter 9 tritt heißes Synthesegas (CO + H₂) bei einer Temperatur von >1000°C aus (abhängig von der Betriebstemperatur des C-Konverters 9). Das aus dem C-Konverter 9 austretende Synthesegas enthält also ebenfalls Wärmeenergie, die direkt oder indirekt über einen in Fig. 1 nicht gezeigten Wärmetauscher beispielsweise zum Vorwärmen des in den Wasser-Eingang 10 eingeleiteten Wassers verwendet werden kann. Bei entsprechender Gestaltung der Betriebsparameter im C-Konverter 9, d.h. einer Temperatur zwischen 1000 und 1200°C, (und der Trennung von Wasserstoff und Kohlenstoff im Kohlenwasserstoffkonverter 3) wird ein Synthesegas erzeugt, bei dem CO und H₂ in einem Verhältnis von 1:1 vorliegen, welches als Wassergas bezeichnet wird. Ohne eine Trennung von Wasserstoff und Kohlenstoff im Kohlenwasserstoffkonverter 3 und entsprechender Gestaltung der Betriebsparameter im C-Konverter 9, d.h. einer Temperatur zwischen 1000 und 1200°C, wird ein Synthesegas erzeugt, bei dem CO und H₂ in einem Verhältnis von ungefähr 1:3 vorliegen

Wie oben erwährt, kann der Kohlenwasserstoffkonverter 3 einen zweiten Kohlenstoffausgang 7 zum Ausleiten von Kohlenstoff aufweisen. Der im Kohlenwasserstoffkonverter 3 erzeugte Kohlenstoff kann - nach einer entsprechenden Trennung (oder auch als C-H₂ Mischung) in unterschiedlichen Anteilen aus dem ersten Kohlenstoffausgang 5 und dem zweiten Kohlenstoffausgang 7 herausgeleitet werden. Der zweite Kohlenstoffausgang 7 wird verwendet, um gegebenenfalls einen Anteil des erzeugten Kohlenstoffes zu entnehmen, der nicht im C-Konverter 9 zur Erzeugung von Synthesegas verwendet wird. Die Größe eines solchen nicht verwendeten Anteils des Kohlenstoffes hängt von der gewünschten Zusammensetzung des Synthesegases ab, welches aus dem C-Konverter 9 ausgeleitet werden soll. Der aus dem zweiten Kohlenstoffausgang 7 entnommene Kohlenstoff kann als Aktivkohle, Graphit, Carbon Black oder andere Modifikation, wie Carbon Cones oder Carbon Discs, entnommen werden. Je nach Form und Qualität des entnommenen Kohlenstoffes kann der entnommene Kohlenstoff als Rohstoff in der chemischen Industrie oder für die Elektronikindustrie verwendet werden. Mögliche Anwendungen sind beispielsweise die Halbleiterherstellung, Reifenherstellung, Tinten, Toner oder ähnliche Produkte. Der vom Kohlenwasserstoffkonverter 3 erzeugte Kohlenstoff ist ein hochreiner Rohstoff, der gut weiterverarbeitet werden kann.

Mit Hilfe des oben dargestellten Verfahrens zur Synthesegas-Erzeugung ist es möglich, den heißen Kohlenstoff aus dem Kohlenwasserstoffkonverter 3 mit warmem bis heißem Wasser im C-Konverter 9 ohne oder wenigstens ohne nennenswerte externe Energiezufuhr zu Synthesegas umzusetzen. Bevorzugt sollten wenigstens 80%, insbesondere wenigstens 90% der zum Erreichen der Umwandlungstemperatur erforderlichen Wärme aus dem Kohlenwasserstoffkonverter 3 stammen.

In Fig. 2 ist eine Anlage 20 zur Erzeugung von Synthesegas gezeigt, welche die oben beschriebenen Elemente der Anlage 1 zur Erzeugung von Synthesegas sowie einen Mischer 21 aufweist, der einen Synthesegaseingang 22 zum Einleiten von Synthesegas und einen H₂-Eingang 23 zum Einleiten von Wasserstoff sowie einen Synthesegasausgang 24 zum Ausleiten von Synthesegas aufweist. Der Synthesegaseingang 22 ist mit dem Synthesegasausgang 12 des C-Konverters 9 verbunden. Der H₂-Eingang 23 des Mischers 21 ist mit dem H₂-Ausgang 6 des Kohlenwasserstoffkonverters 3 verbunden. Wie der Fachmann erkennen kann, wird bei der Ausführung, bei der eine C-H₂ Mischung über den Kohlenstoffausgang 5 in den C-Konverter 9 geleitet wird, automatisch ein Synthesegas mit einem Mischungsverhältnis von CO-H₂ von ungefähr 1:3 erzeugt. In diesem Fall kann ggf. ein Mischer 21 entfallen oder zur Einstellung eines anderen Mischungsverhältnisses eingesetzt werden oder im Mischer CO eingeleitet werden, um den H₂-Anteil des Synthesegases zu vermindern.

Der Mischer 21 kann irgendeine geeignete Vorrichtung zum Vermischen von Gasen sein und zum Beispiel in einem einfachen Fall die Form einer Rohrleitung mit entsprechenden Einlässen und einem Auslaß einnehmen. Mit Hilfe des Mischers 21 und insbesondere über eine Steuerung/Regelung des (zusätzlich) über den H₂-Eingang 23 des Mischers 21 eingeleiteten Wasserstoff und/oder über einen nicht dargestellten Eingang, der mit einer nicht dargestellten CO-Quelle und/oder einer zweiten Synthesegasquelle in Verbindung steht, kann die Mischung des Synthesegases am Synthesegasausgang 24 so beeinflußt werden, dass eine für nachfolgende Prozesse erforderliche Zusammensetzung erreicht wird. Insbesondere kann die zweite Synthesegasquelle ein zweiter C-Konverter 9 sein, der parallel zu einem ersten betrieben wird. Beide C-Konverter 9 könnten von einem gemeinsamen Kohlenwasserstoffkonverters 3 oder auch von separaten Einheiten mit Kohlenstoff und/oder Wasserstoff versorgt werden. Insbesondere kann ein erster mit im Wesentlichen reinem Kohlenstoff (mit vorheriger Trennung des Wasserstoffs) und der zweite mit einem Gemisch aus Kohlenstoff und/oder Wasserstoff versorgt werden. Hierbei würde der erste somit im Wesentlichen Wassergas mit einem Mischungsverhältnis von CO-H₂ von ungefähr 1:1 erzeugen und der zweite ein Synthesegas mit einem Mischungsverhältnis von CO-H₂ von ungefähr 1:3. Ein Zusammenführen beider Synthesegase würde ein Mischungsverhältnis von CO-H₂ von ungefähr 1:2 ergeben, wobei noch überschüssiger Wasserstoff (aus der Trennung vor der Einleitung in den ersten C-Konverter) für eine weitere Erhöhung des Mischungsverhältnisses von CO-H₂ vorhanden wäre.

Für viele Prozesse, beispielsweise die Fischer-Tropsch-Synthese, soll das Verhältnis von Wasserstoff zu CO möglichst hoch sein. Mit Hilfe des Mischers 21 kann ein beliebiges Verhältnis von Wasserstoff zu CO am Synthesegasausgang 24 eingestellt werden, beispielsweise ein Verhältnis von 1:1, was Wassergas entspricht. Es wird in Betracht gezogen, dass nur ein Teil des Synthesegases und/oder nur ein Teil des Wasserstoffes in den Mischer 21 eingeleitet wird, während die nicht in den Mischer eingeleiteten Teile von Synthesegas und Wasserstoff jeweils als reines Gas aus dem Prozeß entnommen werden können. Daher ist es beispielsweise möglich, a) nur Synthesegas zu entnehmen, b) nur Wasserstoff zu entnehmen, c) eine Synthesegasmischung aus CO und Wasserstoff zu entnehmen oder d) einen Strom von Wassergas, einen Strom von Wasserstoff und einen Strom einer Synthesegasmischung (beliebiges Verhältnis von CO zu Wasserstoff) bzw. mehrere Synthesegase mit unterschiedlichem Verhältnis von Kohlenmonoxid zu Wasserstoff zu entnehmen.

Weiterhin weist die Anlage 20 zur Erzeugung von Synthesegas der Fig. 2 einen C-Wärmetauscher 25, einen Synthesegas-Wärmetauscher 26 und einen H₂-Wärmetauscher 27 auf. Der C-Wärmetauscher 25 steht mit der Verbindung 8 zwischen Kohlenwasserstoffkonverter 3 und C-Konverter 9 in thermisch leitendem Kontakt und ist geeignet, der Verbindung ggf. überschüssige Wärme zu entziehen, die nicht für das Erreichen der Umwandlungstemperatur im C-Konverter 9 erforderlich ist, oder Wärme aus anderen Bereichen der Anlage zuzuführen, sofern dies erforderlich ist.

Der Synthesegas-Wärmetauscher 26 steht mit der Verbindung zwischen dem C-Konverter 9 und dem Mischer 21 in thermisch leitendem Kontakt und ist geeignet, der Verbindung und somit dem darin befindlichen heißen Synthesegas überschüssige Wärme zu entziehen. Diese kann zum Beispiel zum Vorheizen des in den C-Konverter 9 eingeleiteten Wassers verwendet werden. Hierfür würde sich insbesondere ein sogenannter Gegenstrom-Wärmetauscher anbieten, wie er in der Technik bekannt ist.

Der H₂-Wärmetauscher 27 steht an der Verbindung zwischen dem Kohlenwasserstoffkonverter 3 und dem Mischer 21 in thermisch leitendem Kontakt und ist geeignet, der Verbindung und somit dem darin befindlichen heißen Wasserstoff überschüssige Wärme zu entziehen. Die mit einem der Wärmetauscher 25, 26 oder 27 abgeführte Wärme kann zum Beheizen anderer Bereiche der Anlage und insbesondere zum Warmhalten des C-Konverters oder zum Vorwärmen des in den C-Konverter eingeleiteten Wassers verwendet werden. Ein Teil der Wärme kann auch beispielsweise über einen Dampferzeuger und eine Dampfturbine oder eine andere geeignete Vorrichtung in Strom umgewandelt werden.

Der Betrieb der Anlage 20 zur Erzeugung von Synthesegas gleicht, was den Betrieb des Kohlenwasserstoffkonverters 3 und des C-Konverters 9 betrifft, dem oben beschriebenen Betrieb der Anlage 1 gemäß Fig. 1. Bei der Anlage 20 zur Erzeugung von Synthesegas wird abhängig von der erwünschten Zusammensetzung des Synthesegases im Mischer 21 ein erwünschtes Mischungsverhältnis von Wasserstoff zu CO eingestellt und am Synthesegasausgang 24 des Mischers 21 ausgeleitet, wobei der Wasserstoff bevorzugt, wie dargestellt, aber nicht zwingend über den Kohlenwasserstoffkonverter 3 zu Verfügung gestellt wird. Es sind hier auch andere Wasserstoffquellen, insbesondere auch ein zweiter Kohlenwasserstoffkonverter 3 denkbar, insbesondere ein Niedertemperatur-Konverter.. Falls nicht die gesamte zur Verfügung stehende Menge an Synthesegas und/oder die gesamte zur Verfügung stehende Menge an H₂ verwendet werden, können die nicht im Mischer zusammengeführten Anteile der jeweiligen Gase, z.B. Wassergas und/oder H₂ jeweils einzeln weiterverarbeitet werden.

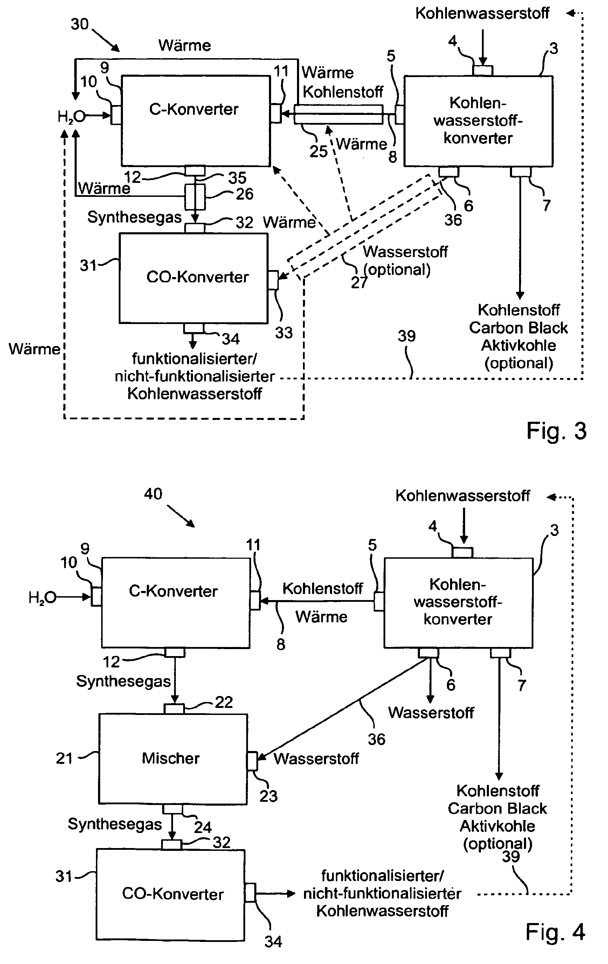

Fig. 3 zeigt eine Anlage 30 zur Erzeugung von synthetischen funktionalisierten und/oder nicht-funktionalisierten Kohlenwasserstoffen, welche eine Anlage 10 zur Erzeugung von Wassergas (wie in Fig. 1 gezeigt) und einen CO-Konverter 31 aufweist. Der Anlagenteil, der der Anlage 1 entspricht wird nicht näher erläutert, um Wiederholungen zu vermeiden. Der CO-Konverter 31 ist stromabwärts zum C-Konverter 9 angeordnet und weist einen Synthesegaseingang 32 zum Einleiten von Synthesegas, einen H₂-Eingang 33 zum Einleiten von Wasserstoff und einen Kohlenwasserstoffausgang 34 zum Ausleiten von synthetischen funktionalisierten und/oder nicht-funktionalisierten Kohlenwasserstoffen auf. Der Synthesegaseingang 32 des CO-Konverters 31 ist mit dem Synthesegasausgang 12 des C-Konverters 9 durch eine Synthesegas-Verbindung 35 verbunden. Der H₂-Eingang 33 des CO-Konverters 31 ist durch eine H₂-Verbindung 36 mit dem H₂-Ausgang 6 des Kohlenwasserstoffkonverters 3 verbunden.

Es sei bemerkt, dass der H₂-Eingang 33 des CO-Konverters 31 und die H₂-Verbindung 36 optionale Elemente sind. Abhängig von der Zusammensetzung des Synthesegases, welches aus dem C-Konverter 9 austritt, und abhängig von den synthetischen funktionalisierten und/oder nicht-funktionalisierten Kohlenwasserstoffen, die im CO-Konverter 31 erzeugt werden sollen, hat das Synthesegas schon beim Austritt aus dem Synthesegasausgang 12 des C-Konverters 9 die richtige Zusammensetzung zur Weiterverarbeitung durch den CO-Konverter 31. In diesem Fall ist das Zuleiten von Wasserstoff über die H₂-Verbindung 36 nicht nötig. Optional kann die H₂-Verbindung 36 aber auch für die Zuleitung eines anderen Stoffes, wie z.B. CO zur Verminderung des H2-Anteils des Synthesegases oder eines Alkens zur Synthese eines Aldehyds (Hydroformylierung), ausgelegt sein.

Die Anlage 30 zur Erzeugung von Kohlenwasserstoffen weist optional auch die in Verbindung mit der Anlage 20 (Fig. 2) beschriebenen Wärmetauscher 25, 26, 27 auf, nämlich den C-Wärmetauscher 25, den Synthesegas-Wärmetauscher 26 und den H₂-Wärmetauscher 27, die in der oben beschriebenen Weise arbeiten (siehe Beschreibung der Fig. 2).

Der CO-Konverter 31 kann ein beliebiger CO-Konverter zur Herstellung von synthetischen, funktionalisierten und/oder nicht-funktionalisierten Kohlenwasserstoffen sein. In der gezeigten Ausführungsform der Fig. 3 ist der CO-Konverter bevorzugt entweder ein Fischer-Tropsch-Konverter, ein Bergius-Pier-Konverter oder ein Pier-Konverter mit einem entsprechenden Katalysator und einer Temperatur und/oder Druck-Steuereinheit.

In einer Ausführungsform weist der CO-Konverter 31 einen Fischer-Tropsch-Konverter auf. Ein Fischer-Tropsch-Konverter wandelt katalytisch ein Synthesegas zu Kohlenwasserstoffen und Wasser um. Dem Fachmann sind verschiedene Ausführungen von Fischer-Tropsch-Reaktoren und Fischer-Tropsch-Verfahren bekannt, die hier nicht im Detail dargestellt werden sollen. Die Hauptreaktionsgleichungen lauten wie folgt:

n CO + (2n+1) H₂ → CnH₂ₙ₊₂ + n H₂O für Alkane

n CO + (2n) H₂ → CₙH₂ₙ + n H₂O für Alkene

n CO + (2n) H₂ CₙH₂ₙ₋₁OH + (n-1) H₂O für Alkohole

Die Fischer-Tropsch-Verfahren können als Hochtemperatur-Verfahren oder als Niedrigtemperatur-Verfahren durchgeführt werden, wobei die Prozeßtemperaturen im Allgemeinen zwischen 200 und 400°C liegen. Bekannte Varianten des Fischer-Tropsch-Verfahrens sind u.a. die Hochlast-Synthese, die Synthol-Synthese und das SMDS-Verfahren der Firma Shell (SMDS = Shell Middle Distillate Synthesis). Durch einen Fischer-Tropsch-Konverter wird typischerweise eine Kohlenwasserstoffverbindung aus Flüssiggasen (Propan, Butan), Benzin, Kerosin (Dieselöl), Weichparaffin, Hartparaffin, Methan- Dieselkraftstoff oder eine Mischung mehrerer derselben erzeugt. Die Fischer-Tropsch-Synthese ist exotherm, wie dem Fachmann bekannt ist. Die Reaktionswärme aus dem Fischer-Tropsch-Verfahren kann mittels eines (in den Figuren nicht gezeigten) Wärmetauschers, beispielsweise zum Vorwärmen von Wasser verwendet werden. Es wird beispielsweise eine zweistufige Vorwärmung des in den C-Konverter 9 eingeleiteten Wassers in Betracht gezogen, wobei zuerst eine Vorwärmung mittels der Abwärme des CO-Konverters 31 (in der Ausführung als Fischer-Tropsch-Konverter) erfolgt und danach eine weitere Erwärmung des Wassers mittels Wärme von einem oder mehreren der Wärmetauscher 25, 26, 27.

In einer alternativen Ausführungsform weist der CO-Konverter 31 einen Bergius-Pier-Konverter oder eine Kombination eines Pier-Konverters mit einem MtL-Konverter (MtL = Methanol-to-Liquid) auf.

In einem Bergius-Pier-Konverter läuft das dem Fachmann wohl bekannte Bergius-Pier-Verfahren ab, bei dem Kohlenwasserstoffe durch Hydrierung von Kohlenstoff mit Wasserstoff in einer exothermen chemischen Reaktion erzeugt werden. Das Spektrum der Ausgangsprodukte aus dem Bergius-Pier-Verfahren hängt von den Reaktionsbedingungen und der Reaktionsführung ab. Es werden hauptsächlich flüssige Endprodukte erhalten, die als Kraftstoffe verwendet werden können, beispielsweise Schwer- und Mittelöle. Bekannte Entwicklungen des Bergius-Pier-Verfahrens sind beispielsweise das Konsol-Verfahren und das H-Coal-Verfahren.

In der oben erwähnten Kombination eines Pier-Konverters mit einem MtL-Konverter wird zunächst Synthesegas nach dem bekannten Pier-Verfahren in Methanol umgewandelt. Der MtL-Konverter ist ein Konverter, in dem Methanol zu Benzin umgewandelt wird. Ein verbreitetes Verfahren ist das MtL-Verfahren der Fa. ExxonMobil bzw. Esso. Eingangsprodukt des MtL-Konverters ist typischerweise Methanol, beispielsweise aus dem Pier-Konverter. Das Ausgangsprodukt, das vom MtL-Konverter erzeugt wird, ist typischerweise Benzin, das zum Betreiben eines Ottomotors geeignet ist.

Zusammenfassend kann gesagt werden, dass in dem CO-Konverter 31, egal nach welchem der oben dargestellten Prinzipien dieser arbeitet, als Endprodukte funktionalisierte und/oder nicht-funktionalisierte Kohlenwasserstoffe synthetisch aus CO und H₂ hergestellt werden können. Die Prozeßwärme, die bei der exothermen Umsetzung im CO-Konverter 31 auftritt, kann wiederum über einen Wärmetauscher entweder zum Beheizen unterschiedlicher Bereiche der Anlage oder zum Erzeugen von Strom verwendet werden, um den Wirkungsgrad der hier beschriebenen Anlagen zu verbessern.

Sofern als Ausgangsprodukt des CO-Konverters 31 eine Mischung von Kohlenwasserstoffen vorliegt, die nach ihrer Auftrennung und Verfeinerung nicht direkt weiterverarbeitet oder als Fertigprodukt profitabel verkauft werden kann, können diese Kohlenwasserstoffe, beispielsweise Methan oder kurzkettige Alkane in den hier beschriebenen Prozeß zurückgeführt werden. Zu diesem Zweck weist die Anlage 30 eine Rückleitungsverbindung 39 auf, mit deren Hilfe ein Teil der synthetisch erzeugten Kohlenwasserstoffe zurück zum Kohlenwasserstoffeingang 4 des Kohlenwasserstoffkonverters 3 geleitet werden können. Je nach Zusammenstellung der zurückgeleiteten, synthetisch erzeugten Kohlenwasserstoffe erfolgt vor der Einleitung in den Kohlenwasserstoffeingang 4 noch eine Aufbereitung bzw. Abtrennung von nicht geeigneten Kohlenwasserstoffen.

Fig. 4 zeigt eine weitere Ausführungsform einer Anlage 40 zur Erzeugung von synthetischen funktionalisierten und/oder nicht-funktionalisierten Kohlenwasserstoffen. Die Anlage 40 weist die oben beschriebene Anlage 20 zur Erzeugung eines Synthesegases sowie einen CO-Konverter 31 auf, wie er oben mit Bezug auf das Ausführungsbeispiel der Fig. 3 beschrieben wurde. Der Synthesegasausgang 24 des Mischers 21 ist mit dem Synthesegaseingang 32 des CO-Konverters 31 verbunden. Der Mischer 21 ist dabei so eingestellt, dass er an seinem Synthesegasausgang 24 ein Synthesegas liefert, welches für die Bedürfnisse des verwendeten CO-Konverters 31 angepaßt ist. Die restlichen Elemente der Anlage 40 sind dieselben, wie oben beschrieben und auch der Betrieb der einzelnen Elemente erfolgt im Wesentlichen in der oben beschriebenen Art und Weise.

Es wird in Betracht gezogen, dass, je nach Größe der Anlage, eine Vielzahl von Kohlenwasserstoffkonvertern nebeneinander betrieben werden kann, um die erwünschte Umwandlungskapazität bereitzustellen. Wie oben erwähnt, können die Kohlenwasserstoffkonverter als Hochtemperatur-Kohlenwasserstoffkonverter und/oder als Niedertemperatur-Kohlenwasserstoffkonverter ausgeführt sein. Ein Hochtemperatur-Kohlenwasserstoffkonverter arbeitet bei Temperaturen von größer 1000°C und ein Niedertemperatur-Kohlenwasserstoffkonverter arbeitet bei Temperaturen zwischen 200 und 1000°C, wobei als zusätzliche Energiequelle zum Beispiel eine Mikrowelleneinheit vorgesehen ist, die Energie direkt in den Kohlenwasserstoff einkoppelt, um eine Aufspaltung in Kohlenstoff und Wasserstoff zu erreichen.

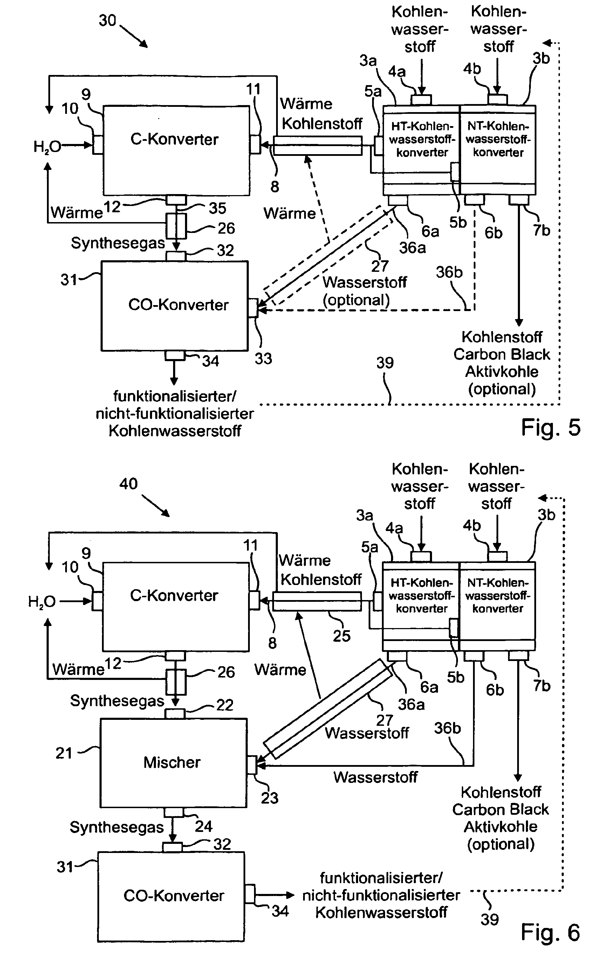

Als Beispiel einer Anlage mit einer Vielzahl von nebeneinander betriebenen Kohlenwasserstoffkonvertern zeigt Fig. 5 eine weitere Ausführung der Anlage 30 zur Erzeugung von synthetischen funktionalisierten und/oder nicht-funktionalisierten Kohlenwasserstoffen. In Fig. 5 werden dieselben Bezugszeichen, wie bei vorherigen Ausführungsformen verwendet, sofern gleiche oder ähnliche Elemente beschrieben werden. Bei der in Fig. 5 gezeigten Ausführung ist statt eines einzelnen der Kohlenwasserstoffkonverters 3 eine Kombination eines Hochtemperatur-Kohlenwasserstoffkonverter 3a und eines Niedertemperatur-Kohlenwasserstoffkonverters 3b vorgesehen.

Der Hochtemperatur-Kohlenwasserstoffkonverter 3a weist einen Kohlenwasserstoffeingang 4a, einen ersten Ausgang 5a zum Ausleiten von Kohlenstoff und einen zweiten Ausgang 6a zum Ausleiten von Wasserstoff auf. Wiederum kann aber auch ein einzelner Ausgang 5a für eine Mischung (insbesondere ein Aerosol) aus Kohlenstoff und Wasserstoff vorgesehen sein. Der erste Ausgang 5a ist über die eine Verbindung 8 mit dem C-Eingang 11 des C-Konverters 9 verbunden. Der optionale zweite Ausgang 6a des Hochtemperatur-Kohlenwasserstoffkonverters 3a ist mit dem H₂-Eingang 33 des CO-Konverters 31 verbunden. Der Hochtemperatur-Kohlenwasserstoffkonverter 3a kann optional einen in der Fig. 5 nicht gezeigten weiteren Ausgang für Kohlenstoff aufweisen.

Der Niedertemperatur-Kohlenwasserstoffkonverter 3b weist einen Prozessraum mit einem Kohlenwasserstoffeingang 4b, einen ersten Ausgang 5b zum Ausleiten von Kohlenstoff, einen zweiten Ausgang 6b zum Ausleiten von Wasserstoff, und einen optionalen, dritten Ausgang 7b zum Ausleiten von Kohlenstoff auf. Bevorzugt weist der Niedertemperatur-Kohlenwasserstoffkonverter 3b eine Trenneinheit auf, um Wasserstoff und Kohlenstoff nach Aufspaltung zu trennen und den jeweiligen Ausgängen zuzuleiten. Der erste Ausgang 5b ist optional über die Verbindung 8 mit dem C-Eingang 11 des C-Konverters 9 verbunden, kann aber auch mit einer Kohlenstoff-Sammeleinheit verbunden sein. Der zweite Ausgang 6b des Niedertemperatur-Kohlenwasserstoffkonverters 3b ist mit dem H₂-Eingang 33 des CO-Konverters 31 verbunden. Der optionale dritte Ausgang 7b ist mit einer Kohlenstoff-Sammeleinheit verbunden, aus der gesammelter Kohlenstoff zum Beispiel als Carbon Black, Aktivkohle oder einer anderen Form entnommen werden kann.

Wie bereits oben bemerkt, sind der H₂-Eingang 33 des CO-Konverters 31 und die H₂-Verbindungen 36a, 36b optionale Elemente, falls das Zuleiten von Wasserstoff über die H₂-Verbindungen 36a, 36b nicht nötig ist.

Der Kohlenwasserstoff, der in den Kohlenwasserstoffeingang 4a und in den Kohlenwasserstoffeingang 4b eingeleitet wird, kann der gleiche Kohlenwasserstoff sein oder es können unterschiedliche Kohlenwasserstoffe sein. In den Kohlenwasserstoffeingang 4a kann Kohlenwasserstoff aus einer ersten Kohlenwasserstoffquelle eingeleitet werden, beispielsweise Erdgas aus einem Erdgasvorrat. In den Kohlenwasserstoffeingang 4b des Niedertemperatur-Kohlenwasserstoffkonverters 3b kann hingegen zum Beispiel funktionalisierter und/oder nicht-funktionalisierter, synthetisch hergestellter Kohlenwasserstoff eingeleitet werden, beispielsweise über die zuvor erwähnte optionale Rückleitungsverbindung 39. Durch die Verwendung von mehreren, parallel betriebenen Kohlenwasserstoffkonvertern 3, 3a, 3b ist die Anlage 30 leichter skalierbar, leichter steuerbar und es können unterschiedliche Arten von Kohlenstoff hergestellt werden.

Darüber hinaus kann zum Beispiel mit Vorteil der Hochtemperatur-Kohlenwasserstoffkonverter 3a verwendet werden, um "heißen" Kohlenstoff vorzugsweise mit einer Temperatur über 1000°C für die Umwandlung im C-Konverter 9 zu erzeugen. Hierbei kann der Hochtemperatur-Kohlenwasserstoffkonverter 3a insbesondere ohne eine Trenneinheit auskommen, da die durch die Aufspaltung gewonnene C-H₂ Mischung direkt in den C-Konverter 9 eingeleitet werden kann. In diesem Fall gibt der C-Konverter 9 dann am Ausgang zum Beispiel ein Synthesegas mit einem C-H₂ Mischungsverhältnis von ungefähr 1:3 aus.

Der Niedertemperatur-Kohlenwasserstoffkonverter 3b wird hingegen primär zur Erzeugung von zusätzlichem Wasserstoff für die Erzeugung eines Synthesegases oder einer C-H₂ Mischung mit einem C-H₂ Mischungsverhältnis von größer 1:3 für den CO-Konverter 31 bereitstellen zu können. Da hier kein Wärmetransfer vom Niedertemperatur-Kohlenwasserstoffkonverter 3b zu einem nachfolgenden Prozeß erforderlich ist, kann er Vorteilhaft mit Temperaturen unter 1000°C und bevorzugt bei der niedrigsten möglichen Temperatur betrieben werden.

Im Betrieb der Anlage 30 kann somit ein Teil des in den Kohlenwasserstoffkonvertern 3a, 3b erzeugten Kohlenstoffes (bevorzugt der aus dem Hochtemperatur-Kohlenwasserstoffkonverter 3a) in den C-Konverter 9 eingeleitet werden, während ein anderer Teil (bevorzugt der aus dem Niedertemperatur-Kohlenwasserstoffkonverter 3b) als Grundstoff für die Erzeugung von weiteren Produkten aus dem Verfahren ausgeleitet werden kann. Solche Produkte sind beispielsweise Carbon Black bzw. Industrieruß, Aktivkohle, spezielle Modifikationen des Kohlenstoffs wie Carbon Discs und Carbon Cones usw., was als schwarzer, pulverförmiger Feststoff vorliegt. Dieser Kohlenstoff ist ein wichtiges technisches Produkt, welches beispielsweise als Füllstoff in der Gummiindustrie, als Pigmentruß für Druckfarben, Tuschen und Lacke oder als Ausgangsstoff zur Herstellung von elektrischen Bauteilen verwendet wird, beispielsweise für Zink-Kohle-Batterien und zur Herstellung von Kathoden oder Anoden. Gegebenenfalls überschüssiger Wasserstoff kann für die chemische Industrie ausgeleitet oder auch zur Erzeugung von Strom (durch verbrennen oder mittels Brennstoffzelle) verwendet werden, wobei der Niedertemperatur-Kohlenwasserstoffkonverter 3b bevorzugt so betrieben wird, dass er nur den erforderlichen zusätzlichen Wasserstoff bereitstellt.

Fig. 6 zeigt eine alternative Ausführung der oben beschriebenen Anlage 40 zur Erzeugung von synthetischen, funktionalisierten und/oder nicht-funktionalisierten Kohlenwasserstoffen, bei der ebenfalls eine Vielzahl von parallel betriebenen Hochtemperatur- und/oder Niedertemperatur-Kohlenwasserstoffkonvertern vorgesehen ist. Die in Fig. 6 gezeigte Anlage 40 zur Erzeugung von Kohlenwasserstoffen unterscheidet sich von der in Fig. 5 gezeigten Anlage 30 dadurch, dass vor dem CO-Konverter 31 ein Mischer 21 vorgeschaltet ist, der ein speziell für den CO-Konverter 31 zugeschnittenes Synthesegas zusammenmischt und an den CO-Konverter 31 liefert. Die in Fig. 6 dargestellten Elemente wurden oben bereits zuvor beschrieben und arbeiten auch nach den oben beschriebenen Prinzipien. Daher wird hier auf eine erneute detaillierte Beschreibung verzichtet, um Wiederholungen zu vermeiden.

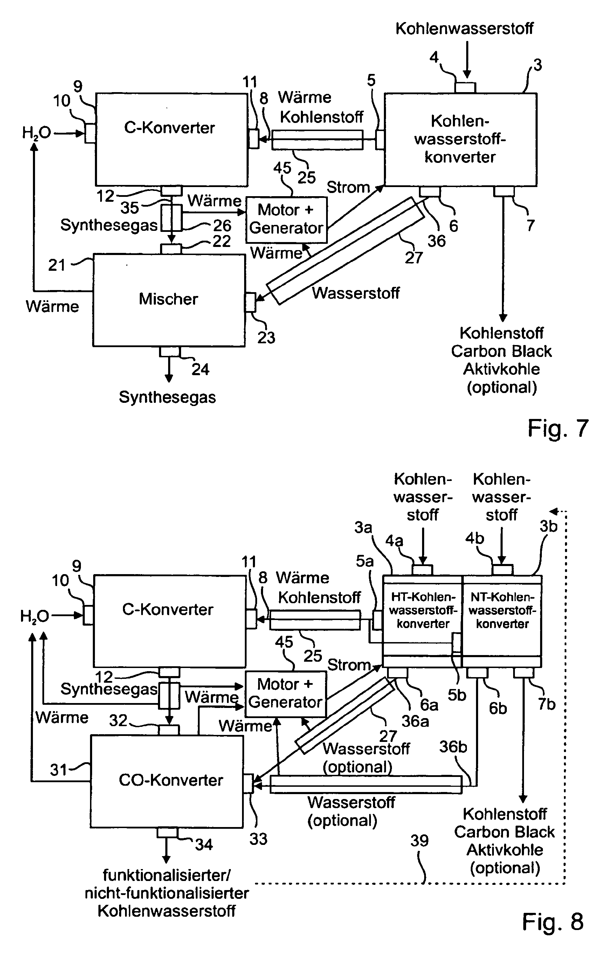

Die Fig. 7 und 8 zeigen Ausführungsformen der Anlagen 20 und 30, die einen C-Wärmetauscher 25, einen Synthesegas-Wärmetauscher 26 und H₂-Wärmetauscher 27 aufweisen, welche jeweils mit einer Motor/Generator-Anordnung 45 verbunden sind. Die Motor/Generator-Anordnung 45 ist geeignet, aus überschüssiger Wärme von unterschiedlichen Stellen der Anlage zumindest teilweise elektrischen Strom zu erzeugen, welcher entweder in ein allgemeines Stromnetz eingespeist werden kann oder zum Betrieb der Anlage 20, 30 insbesondere des/der Kohlenwasserstoffkonverter verwendet werden kann. Weiter kann die Motor/Generator-Anordnung 45 mit einem in Fig. 8 nicht gezeigten Wärmetauscher am CO-Konverter 31 verbunden sein, der die Wärme ableitet, die bei dem exothermen Umwandlungsprozeß entsteht, der im CO-Konverter 31 abläuft. So kann einerseits der CO-Konverter 31 in gesteuerter oder geregelter Weise gekühlt werden, was für die Prozeßführung vorteilhaft ist, und andererseits elektrischer Strom erzeugt werden. Die Motor/Generator-Anordnung 45 kann irgendeine Vorrichtung sein, welche zur Verstromung von Wärmeenergie geeignet ist, beispielsweise eine Kombination aus einer Dampfturbine und einem Generator oder einem Kolbenmotor und einem Generator.

Die Motor/Generator-Anordnung 45 verstromt im Betrieb den Anteil der überschüssigen Wärme in der Anlage, d.h. die Wärme die nicht zum Erreichen der Kohlenstoff-Wasser Umwandlung verwendet wird.

Die Motor/Generator-Anordnung 45 und die Wärmetauscher 25, 26 und 27 sind optionale Elemente, die bei allen oben genannten Anlagen eingesetzt werden können. Auch der aus dem jeweiligen zweiten Kohlenstoffausgang 7, 7a, 7b ausgeleitete Kohlenstoff enthält aufgrund der Betriebstemperatur im jeweiligen Kohlenwasserstoffkonverter 3, 3a, 3b eine beträchtliche Wärmeenergie. Abhängig von der erwünschten Temperatur des ausgeleiteten Kohlenstoffs kann ein Großteil dieser Wärmeenergie mittels in den Fig. nicht gezeigten Wärmetauschern abgeleitet werden und in den hier beschriebenen Verfahren weiterverwendet werden und/oder über die Motor/Generator-Anordnung 45 in Strom umgewandelt werden.

Bei den Anlagen 30 und 40 zur Erzeugung von synthetischen funktionalen und/oder nicht-funktionalen Kohlenwasserstoffen erfolgt die Abkühlung des Wasserstoffes aus dem Kohlenwasserstoffkonverter 3, 3a, 3b und/oder des Synthesegases aus dem C-Konverter 9 nur soweit, dass die Betriebstemperatur des CO-Konverters 31 nicht unterschritten wird. Die Betriebstemperatur des CO-Konverters 31 liegt üblicherweise bei 200 bis 400°C, abhängig vom eingesetzten Verfahren.

Der Kohlenwasserstoffkonverter 3 kann bei allen oben beschriebenen Anlagen ein Hochtemperatur-Reaktor sein, welcher bei einer Temperatur von mehr als 1000°C arbeitet (z.B. ein Hochtemperatur-Kvaerner-Reaktor), oder ein Niedertemperatur-Reaktor, der bei einer Temperatur zwischen 200°C und 1000°C arbeitet (z.B. ein Niedertemperatur-Kvaerner-Reaktor). Ein derzeit erprobter Niedertemperatur-Reaktor arbeitet bei Temperaturen von 400 bis 900°C. Im Fall eines Niedertemperatur-Reaktor, der bei Temperaturen von 200 bis 900°C arbeitet, wird in Betracht gezogen, dass der eingeleitete Kohlenstoff in der Verbindung 8 zwischen Kohlenwasserstoffkonverter 3 und C-Konverter 9 vorgeheizt wird, da der C-Konverter 9 bei Temperaturen von 800 bis 1700°C und vorzugsweise 1000 bis 1200°C arbeitet. Aus den Fig. 7 und 8 wird weiter deutlich, dass eine Kombination von Hochtemperatur- und/oder Niedertemperatur-Kohlenwasserstoffkonvertern bei allen oben beschriebenen Anlagen 1, 20, 30 und 40 verwendet werden kann.

Ebenso kann bei allen oben beschriebenen Anlagen 1, 20, 30 und 40 ein Teil des im Kohlenwasserstoffkonverter 3, 3a, 3b erzeugten Kohlenstoffes als Carbon Black, Aktivkohle oder sonstiger Rohstoff entnommen werden, sofern dieser Kohlenstoff nicht im C-Konverter 9 der Anlage 1, 20, 30, 40 umgesetzt wird. Weiterhin sei bemerkt, dass bei allen oben beschriebenen Anlagen eine Vielzahl von C-Konvertern 9 vorgesehen sein kann, die jeweils einen Teil des erzeugten Kohlenstoffes unter Zuleitung von Wasser zu Synthesegas umwandeln. Weiter kann optional bei allen oben beschriebenen Anlagen 30 und 40 eine Rückleitung von nicht erwünschten Anteilen der im CO-Konverter 31 synthetisch erzeugten funktionalisierten und/oder nicht-funktionalisierten Kohlenwasserstoffe zum Kohlenwasserstoffeingang 4, 4a, 4b des Kohlenwasserstoffkonverters 3 erfolgen.

In den Anlagen 1, 20, 30, 40 und bei den Verfahren zur Erzeugung von Synthesegas und/oder synthetischen funktionalisierten und/oder nicht-funktionalisierten Kohlenwasserstoffen kann überschüssiger Wasserstoff anfallen. Beispielsweise bleibt bei einem Synthesegas mit niedrigem H₂-Anteil noch Wasserstoff übrig, und abhängig von den synthetischen Kohlenwasserstoffen, die im CO-Konverter 31 erzeugt werden sollen, ist das Zuleiten von Wasserstoff in den Mischer 21 oder den CO-Konverter 31 nicht nötig. In diesen Fällen kann der überschüssige Wasserstoff auch direkt mittels Verbrennung oder in einer Brennstoffzelle in Strom umgewandelt werden. Dadurch kommt das Verfahren weitgehend ohne externe Stromzufuhr aus. Dies ist besonders vorteilhaft bei Anlagen, die an entlegenen Orten betrieben werden, an denen keine leistungsfähige allgemeine Stromversorgung zur Verfügung steht. Weiterhin sei bemerkt, dass ebenfalls ein Teil des im Kohlenwasserstoffkonverter 3 erzeugten Wasserstoffes direkt aus dem Verfahren ausgeleitet und als Rohstoff vermarktet werden kann.

In allen Anlagen können die Ströme von Kohlenstoff, Synthesegas und Wasserstoff bzw. externes CO zwischen den Konvertern 3, 9, 31 und dem Mischer durch Ventilelemente, Klappen, Schieber o.ä. gesteuert werden. Insbesondere wird in Betracht gezogen, dass der Zufluss von Synthesegas und Wasserstoff bzw. CO in den CO-Konverter 31 durch Ventile gesteuert werden kann. Eine Vermischung von Synthesegas und Wasserstoff bzw. CO in einem erwünschten Verhältnis läuft dann direkt im CO-Konverter 31 ab.

In allen oben dargestellten Anlagen kann der CO-Konverter 31 aus einer Vielzahl von CO-Konvertern bestehen (in den Zeichnungen nicht gezeigt), wobei die Gesamtmenge von im Kohlenwasserstoffkonverter 3, 3a, 3b erzeugten und abgetrennten Wasserstoffs und des im C-Konverter 9 erzeugten Synthesegases beliebig auf die Vielzahl von CO-Konvertern aufgeteilt werden kann.

Die einzelnen CO-Konverter haben eine der oben beschriebenen Bauarten und Betriebsweisen. Die CO-Konverter können die gleiche Bauart und Betriebsweise aufweisen, oder verschiedene Bauarten und Betriebsweisen. In einer Ausführung mit unterschiedlichen CO-Konvertern können die einzelnen CO-Konverter jeweils mit unterschiedlich zusammengesetztem Synthesegas betrieben werden und unterschiedliche Produkte liefern.

Es folgen zur weiteren Veranschaulichung einige Anwendungsbeispiele:

### Beispiel 1:

Nimmt man 1 Teil Methan und spaltet es im Kohlenwasserstoffkonverter, so erhält man einen Teil Kohlenstoff und zwei Teile Wasserstoff. Der Kohlenstoff wird im C-Konverter mit einem Teil Wasser zu einem Teil Kohlenmonoxid und einem Teil Wasserstoff umgesetzt. Nach Zufügung von 1,1 Teilen Wasserstoff kann das Synthesegas im CO-Konverter zu Paraffin umgesetzt werden. Es steht dann noch genügend Wasserstoff zur Verfügung, um das Paraffin in einem weiteren Schritt zu Diesel, Ottokraftstoff oder Kerosin zu spalten.

### Beispiel 2:

Nimmt man ein Teil Propan (Butan) und spaltet es im Kohlenwasserstoffkonverter, so erhält man 3 (4) Teile Kohlenstoff und 4 (5) Teile Wasserstoff. Der Kohlenstoff wird im C-Konverter mit 3 (4) Teilen Wasser zu einem Gemisch aus 3 (4) Teilen Kohlenmonoxid und 3 (4) Teilen Wasserstoff umgesetzt. Fügt man dem Synthesegas weitere 3,3 (4,4) Teile Wasserstoff zu, so kann es im CO-Konverter zu Paraffin umgesetzt werden. Die Menge des verbliebenen Wasserstoffs ist in beiden Fällen gerade ausreichend, um das Paraffin in einem weiteren Schritt zu Diesel, Ottokraftstoff oder Kerosin zu spalten.

### Beispiel 3:

Nimmt man ein Teil Schweröl (z.B. C₂₀H₄₂) und spaltet es im Kohlenwasserstoffkonverter, so erhält man 20 Teile Kohlenstoff und 21 Teile Wasserstoff. Der Kohlenstoff wird im C-Konverter mit 20 Teilen Wasser zu 20 Teilen Kohlenmonoxid und 20 Teilen Wasserstoff umgesetzt. Nach Hinzufügen der 21 Teile Wasserstoff kann das Synthesegas in einem anderen CO-Konverter zu 20 Teilen Methanol umgesetzt werden.

Da bei den hier beschriebenen Verfahren der Wasserstoff, der bei der Spaltung von Kohlenwasserstoffen im Köhlenwasserstoffkonverter 3 erzeugt wird, von dem ebenfalls dort erzeugten Kohlenstoff getrennt wird, läßt sich der abgetrennte Wasserstoff nach erfolgter Erzeugung eines wasserstoffarmen Synthesegases eben diesem wasserstoffarmen Synthesegas in jedem beliebigen Verhältnis wieder zuführen. Eine Bandbreite des Verhältnisses von Wasserstoff zu CO von 1,0 bis 3,0 ist so möglich. Durch partielle Oxidation von überschüssigem Kohlenstoff ist ein Verhältnis von < 1,0 erreichbar, und durch Nichtverwenden von überschüssigem Kohlenstoff ist ein Verhältnis von > 3,0 erreichbar.

Die Erfindung wurde anhand bevorzugter Ausführungsbeispiele beschrieben, wobei die einzelnen Merkmale der beschriebenen Ausführungsbeispiele frei miteinander kombiniert werden können und/oder ausgetauscht werden können, sofern sie kompatibel sind. Ebenso können einzelne Merkmale der beschriebenen Ausführungsbeispiele weggelassen werden, sofern sie nicht zwingend notwendig sind. In einer besonders einfachen Ausgestaltung einer Anlage zur Erzeugung von synthetischen funktionalen und/oder nicht-funktionalen Kohlenwasserstoffen kann der C-Konverter zum Beispiel als einfache Rohrleitung (zum Beispiel ein Ausgangsrohr eines keine Trenneinheit aufweisenden Hochtemperatur-Kohlenwasserstoffkonverters) ausgebildet sein in das ein Wassereinlaß mündet. Dabei sollte der Wassereinlaß so in die Rohrleitung einmünden, dass eine gute Durchmischung der jeweiligen Medienströme erreicht wird. Die Rohrleitung sollte von einer Isolierung umgeben sein und könnte beispielsweise im Bereich eines Eingangs mit einer Heizeinheit in Verbindung stehen, um die Rohrleitung insbesondere zu Beginn des Betriebs auf eine Betriebstemperatur vorzuwärmen. Weiter stromabwärts könnte die Rohrleitung wiederum mit einem Wärmetauscher in Verbindung stehen, der überschüssige Wärme ableiten und gegebenenfalls zum Wärmen anderer Bereiche der Anlage und/oder zur Erzeugung von Strom einsetzen kann. In die Rohrleitung kann zusätzlich (zum Beispiel stromabwärts des Wärmetauschers) eine Zuleitung für Wasserstoff aufweisen, sodass dieselbe Rohrleitung nicht nur die Funktion eines C-Konverters sondern auch eines Mischers zum Erzeugen eines Synthesegases mit einem bestimmten Mischungsverhältnis übernehmen kann. Die Zuleitung für Wasserstoff kann zum Beispiel von einem Wasserstoffausgang eines Niedertemperatur-Kohlenwasserstoffkonverters (mit Trenneinheit) stammen. Ein Ausgangsende der Rohrleitung, an dem ein Synthesegas mit einem vorbestimmten Mischungsverhältnis ausgegeben werden kann, könnte dann in einem CO-Konverter enden.

## Patentansprüche

1. Verfahren zum Erzeugen von Synthesegas, welches folgende Schritte aufweist:
Aufspalten eines Kohlenwasserstoff enthaltenden Fluids zu Kohlenstoff und Wasserstoff durch einen Energieeintrag, der wenigstens teilweise durch Wärme geleistet wird, wobei der Kohlenstoff und der Wasserstoff nach der Aufspaltung eine Temperatur von wenigstens 200°C aufweist;
In-Kontakt-Bringen von Wasser mit wenigstens einem Teil des Kohlenstoffes, der aus der Aufspaltung gewonnen wurde, bei einer Temperatur von 800 bis 1700°C, wobei sich der durch die Aufspaltung gewonnene Kohlenstoff beim In-Kontakt-Bringen mit dem Wasser um höchstens 50% in °C hinsichtlich seiner Temperatur nach der Aufspaltung abgekühlt hat;
Umwandeln wenigstens eines Teils des Wassers und des durch die Aufspaltung gewonnenen Kohlenstoffes in ein Synthesegas;
wobei der durch die Aufspaltung gewonnene Kohlenstoff und der durch die Aufspaltung gewonnene Wasserstoff gemeinsam mit dem Wasser in Kontakt gebracht werden.

2. Verfahren zum Erzeugen von Synthesegas, welches folgende Schritte aufweist:
Aufspalten eines Kohlenwasserstoff enthaltenden Fluids zu Kohlenstoff und Wasserstoff durch einen Energieeintrag, der wenigstens teilweise durch Wärme geleistet wird, wobei der Kohlenstoff und der Wasserstoff nach der Aufspaltung eine Temperatur von wenigstens 200°C aufweist;
In-Kontakt-Bringen von Wasser mit wenigstens einem Teil des Kohlenstoffes, der aus der Aufspaltung gewonnen wurde, bei einer Temperatur von 800 bis 1700°C, wobei sich der durch die Aufspaltung gewonnene Kohlenstoff beim In-Kontakt-Bringen mit dem Wasser um höchstens 50% in °C hinsichtlich seiner Temperatur nach der Aufspaltung abgekühlt hat;
Umwandeln wenigstens eines Teils des Wassers und des durch die Aufspaltung gewonnenen Kohlenstoffes in ein Synthesegas;
wobei der durch die Aufspaltung gewonnene Wasserstoff wenigstens teilweise von dem Kohlenstoff getrennt wird, und zwar vor dem In-Kontakt-Bringen mit dem Wasser, und
wobei dem durch die Umwandlung entstehenden Synthesegas der abgetrennte Wasserstoff zumindest teilweise beigemischt wird.

3. Verfahren zum Erzeugen von Synthesegas nach Anspruch 1 oder 2,
wobei die Aufspaltung bei einer Temperatur über 1000°C erfolgt und der Kohlenstoff bei einer Temperatur von wenigstens 1000°C, insbesondere bei einer Temperatur zwischen 1000°C und 1200°C mit dem Wasser in Kontakt gebracht wird;
wobei die erforderliche Wärme zum Erreichen der Temperatur von 800 bis 1700°C für die Umwandlung bevorzugt im Wesentlichen vollständig durch die Wärme, die für die Aufspaltung des Kohlenwasserstoff enthaltenden Fluids eingebracht wird, bereitgestellt wird.

4. Verfahren zum Erzeugen von Synthesegas nach einem der vorhergehenden Ansprüche, wobei wenigstens ein Teil der Wärme wenigstens eines Teils des durch die Aufspaltung gewonnenen Kohlenstoffs und/oder Wasserstoffes und/oder des Synthesegases zum Erwärmen des Wassers vor dem In-Kontakt-Bringen mit dem Kohlenstoff und/oder zum Erwärmen eines Prozessraums in dem das Wasser mit dem Kohlenstoff in Kontakt gebracht wird, und/oder zur Erzeugung von elektrischem Strom verwendet wird, der insbesondere als Energieträger für den Energieeintrag für das Aufspalten des Kohlenwasserstoff enthaltenden Fluids bereitgestellt wird.

5. Verfahren zum Erzeugen von Synthesegas nach einem der vorhergehenden Ansprüche, wobei der Energieeintrag zum Aufspalten des Kohlenwasserstoff enthaltenden Fluids primär über ein Plasma insbesondere in einem Kvaerner-Reaktor erfolgt.

6. Verfahren zum Erzeugen von Synthesegas, nach Anspruch 5, wobei der durch die Aufspaltung gewonnene Kohlenstoff und der durch die Aufspaltung gewonnene Wasserstoff als Aerosol mit dem Wasser in Kontakt gebracht werden.

7. Verfahren zum Erzeugen von synthetischen, funktionalisierten und/oder nicht-funktionalisierten Kohlenwasserstoffen, bei dem zunächst ein Synthesegas nach einem der Ansprüche 1 bis 6 erzeugt und dieses mit einem geeigneten Katalysator in Kontakt gebracht wird, um eine Umwandlung des Synthesegases in synthetische, funktionalisierte und/oder nicht-funktionalisierte Kohlenwasserstoffe zu bewirken, wobei die Temperatur des Katalysators und oder des Synthesegases auf einen vorbestimmten Temperaturbereich gesteuert oder geregelt wird.

8. Verfahren zum Erzeugen von synthetischen, funktionalisierten und/oder nicht-funktionalisierten Kohlenwasserstoffen nach Anspruch 7, wobei die Umwandlung des Synthesegases mittels eines der folgenden Verfahren erfolgt: Fischer-Tropsch-Verfahren, SMDS-Verfahren, Bergius-Pier-Verfahren, Pier-Verfahren oder eine Kombination aus einem Pier-Verfahren mit einem MtL-Verfahren.

9. Vorrichtung zum Erzeugen eines Synthesegases, die folgendes aufweist:
einen Kohlenwasserstoffkonverter zum Aufspalten eines Kohlenwasserstoff enthaltenden Fluids in Kohlenstoff und Wasserstoff, der wenigstens einen Prozessraum mit wenigstens einem Eingang für ein Kohlenwasserstoff enthaltendes Fluid und wenigstens einem Ausgang für Kohlenstoff und/oder Wasserstoff und wenigstens eine Einheit zum Einbringen von Energie in den Prozessraum, die wenigstens teilweise aus Wärme besteht, aufweist;
einen C-Konverter zur Umwandlung von Wasser und Kohlenstoff, der wenigstens einen weiteren Prozessraum mit wenigstens einem Eingang für Wasser, wenigstens einem Eingang für wenigstens Kohlenstoff und wenigstens einem Ausgang aufweist, wobei der Eingang für wenigstens Kohlenstoff direkt mit dem wenigstens einem Ausgang des Kohlenwasserstoffkonverters verbunden ist;
wobei der Kohlenwasserstoffkonverter, sein Ausgang, der der C-Konverter und sein Eingang so ausgebildet sind, dass im Kohlenwasserstoffkonverter ausgebildeter Kohlenstoff und Wasserstoff gemeinsam in den C-Konverter geleitet werden.

10. Vorrichtung zum Erzeugen eines Synthesegases, die folgendes aufweist:
einen Kohlenwasserstoffkonverter zum Aufspalten eines Kohlenwasserstoff enthaltenden Fluids in Kohlenstoff und Wasserstoff, der wenigstens einen Prozessraum mit wenigstens einem Eingang für ein Kohlenwasserstoff enthaltendes Fluid und wenigstens einem Ausgang für Kohlenstoff und/oder Wasserstoff und wenigstens eine Einheit zum Einbringen von Energie in den Prozessraum, die wenigstens teilweise aus Wärme besteht, aufweist;
einen C-Konverter zur Umwandlung von Wasser und Kohlenstoff, der wenigstens einen weiteren Prozessraum mit wenigstens einem Eingang für Wasser, wenigstens einem Eingang für wenigstens Kohlenstoff und wenigstens einem Ausgang aufweist, wobei der Eingang für wenigstens Kohlenstoff direkt mit dem wenigstens einem Ausgang des Kohlenwasserstoffkonverters verbunden ist;
wenigstens eine Trenneinheit zum Trennen des durch die Aufspaltung entstandenen Kohlenstoffs und des Wasserstoffs, die getrennte Ausgänge für die getrennten Stoffe aus der Trenneinheit aufweist, wobei der Ausgang für Kohlenstoff mit dem C-Konverter verbunden ist, wobei eine separate Zuleitung für den Wasserstoff aus der Trenneinheit in einen stromabwärts befindlichen Mischraum vorgesehen ist.

11. Vorrichtung zum Erzeugen eines Synthesegases nach Anspruch 9 oder 10, wobei die wenigstens eine Einheit zum Einbringen von Energie in den Prozessraum so ausgebildet ist, dass sie wenigstens lokal Temperaturen über 1000°C erzeugen kann; und/oder
wobei die wenigstens eine Einheit zum Einbringen von Energie in den Prozessraum eine Plasmaeinheit in einem Kvaerner-Reaktor aufweist.

12. Vorrichtung zum Erzeugen eines Synthesegases nach einem der Ansprüche 9 bis 11, wobei der Prozessraum des C-Konverters durch ein Auslaßrohr des Kohlenwasserstoffkonverters gebildet wird, das mit einer Einspeisung für Wasser verbunden ist.

13. Vorrichtung zum Erzeugen eines Synthesegases nach Anspruch 11, wobei der Kohlenwasserstoffkonverter geeignet ist, ein Aerosol aus Kohlenstoff und Wasserstoff zu erzeugen.

14. Vorrichtung zum Erzeugen eines Synthesegases nach einem der Ansprüche 9 bis 13, mit wenigstens einem weiteren Kohlenwasserstoffkonverter zum Aufspalten eines Kohlenwasserstoff enthaltenden Fluids in Kohlenstoff und Wasserstoff, der folgendes aufweist:
Wenigstens einen Prozessraum mit wenigstens einem Eingang für das Kohlenwasserstoff enthaltende Fluid,
wenigstens eine Einheit zum Einbringen von Energie in den Prozessraum, die wenigstens teilweise aus Wärme besteht,
eine Trenneinheit zum Trennen des durch die Aufspaltung entstandenen Kohlenstoffs und des Wasserstoffs mit getrennten Ausgängen für Kohlenstoff und Wasserstoff, wobei der Ausgang für Wasserstoff mit der separaten Zuleitung für Wasserstoff verbunden ist;
wobei der wenigstens eine weitere Kohlenwasserstoffkonverter des Typs ist, der eine Aufspaltung bei Temperaturen unter 1000°C, insbesondere unter 600°C, mittels eines Mikrowellenplasmas bewirkt.

15. Vorrichtung zum Umwandeln eines Synthesegases in synthetische, funktionalisierte und/oder nicht-funktionalisierte Kohlenwasserstoffe, die folgendes aufweist:
eine Vorrichtung nach einem der Ansprüche 9 bis 14; und
einen CO-Konverter mit einem Prozessraum in dem ein Katalysator angeordnet ist und Mittel zum Leiten des Synthesegases in Kontakt mit dem Katalysator, und eine Steuereinheit zum Steuern oder Regeln der Temperatur des Katalysators und/oder des Synthesegases auf eine vorbestimmte Temperatur.

16. Vorrichtung nach Anspruch 15, wobei der CO-Konverter einen der Folgenden Konverter aufweist: einen Fischer-Tropsch-Konverter, einen SMDS-Konverter, einen Bergius-Pier-Konverter, einen Pier-Konverter oder eine Kombination eines Pier-Konverters mit einen MtL-Konverter.

## Claims

1. A method for generating synthesis gas comprising the following steps:
decomposing a hydrocarbon containing fluid into carbon and hydrogen by means of introduction of energy, the energy at least partially being provided by heat, wherein the carbon and the hydrogen have a temperature of at least 200°C after the decomposing step;
bringing water into contact with at least a portion of the carbon generated by the decomposing step at a temperature between 800 and 1700°C, wherein upon bringing the carbon into contact with water, the carbon obtained by the decomposing step has cooled down by no more than 50% in °C with respect to its temperature after the decomposing step;
transforming at least a portion of the water and the carbon obtained by the decomposing step into synthesis gas;
wherein the carbon obtained by the decomposing step and the hydrogen obtained by the decomposing step are jointly brought into contact with the water.

2. A method for generating synthesis gas comprising the following steps:
decomposing a hydrocarbon containing fluid into carbon and hydrogen by means of introduction of energy, the energy at least partially being provided by heat, wherein the carbon and the hydrogen have a temperature of at least 200°C after the decomposing step;
bringing water into contact with at least a portion of the carbon generated by the decomposing step at a temperature between 800 and 1700°C, wherein upon bringing the carbon into contact with water, the carbon obtained by the decomposing step has cooled down by no more than 50% in °C with respect to its temperature after the decomposing step;
transforming at least a portion of the water and the carbon obtained by the decomposing step into synthesis gas;
wherein the carbon obtained by the decomposing step is separated at least partially from the hydrogen obtained by the decomposing step prior to the step of bringing the carbon into contact with the water, and
wherein at least a portion of the separated hydrogen is added to the synthesis gas generated by the transformation.

3. The method for generating synthesis gas according to claim 1 or 2, wherein the decomposing step takes place at a temperature above 1000°C; and wherein the carbon is brought into contact with the water at a temperature of at least 1000°C, particularly at a temperature between 1000°C and 1200°C; and
wherein preferably the heat required to reach the temperature of between 800 and 1700°C for the transformation originates essentially completely from the heat that is provided for decomposing the hydrocarbon containing fluid.

4. The method for generating synthesis gas according to one of the preceding claims, wherein at least a portion of the heat of at least a portion of the carbon obtained by the decomposing step and/or of at least a portion of the hydrogen obtained by the decomposing step and/or of at least a portion of the synthesis gas is used to heat the water prior to bringing the water in contact with carbon and/or is used to heat the process chamber, in which the water is brought into contact with the carbon and/or is used for generating electricity, wherein the electricity particularly may be provided as an energy carrier for introducing energy for decomposing the hydrocarbon containing fluid.

5. The method for generating synthesis gas according to one of the preceding claims, wherein the energy for decomposing the hydrocarbon containing fluid is primarily introduced by means of a plasma, and wherein the decomposing step is preferably performed in a Kvaerner reactor.

6. The method for generating synthesis gas according to claim 5, wherein the carbon generated by decomposing and the hydrogen generated by decomposing are brought into contact with water in form of an aerosol.

7. A method for generating synthetic functionalised and/or non-functionalised hydrocarbons, wherein at first a synthesis gas is generated according to one of the claims 1 to 8, and wherein the synthesis gas is brought into contact with a suitable catalyst in order to cause the transformation of the synthesis gas to synthetic functionalised and/or non-functionalised hydrocarbons, wherein the temperature of the catalyst and/or the synthesis gas is open-loop controlled or close-loop regulated to a predetermined range of temperatures.

8. The method for generating synthetic functionalised and/or non-functionalised hydrocarbons according to claim 7, wherein the transformation of the synthesis gas is carried out by means of one of the following processes: a Fischer-Tropsch process, a SMDS process, a Bergius-Pier process, a Pier process, or a combination of a Pier process and a MtL process.

9. An apparatus for generating synthesis gas comprising:
a hydrocarbon converter for decomposing a hydrocarbon containing fluid into carbon and hydrogen, wherein the hydrocarbon converter comprises at least one process chamber having at least one inlet for a hydrocarbon containing fluid and at least one outlet for carbon and/or hydrogen and at least one unit for introducing energy into the process chamber, the energy consisting at least partially of heat;
a C converter for transformation of water and carbon, the C converter comprising at least one further process chamber having at least one inlet for water, at least one inlet for at least carbon and at least one outlet, wherein the inlet for at least carbon is directly connected to the at least one outlet of the hydrocarbon converter;
wherein the hydrocarbon converter, the at least one outlet of the hydrocarbon converter the C converter and the at least one inlet of the C converter are adapted to concurrently direct said carbon and said hydrogen produced in the hydrocarbon converter into the C converter.

10. An apparatus for generating synthesis gas comprising:
a hydrocarbon converter for decomposing a hydrocarbon containing fluid into carbon and hydrogen, wherein the hydrocarbon converter comprises at least one process chamber having at least one inlet for a hydrocarbon containing fluid and at least one outlet for carbon and/or hydrogen and at least one unit for introducing energy into the process chamber, the energy consisting at least partially of heat;
a C converter for transformation of water and carbon, the C converter comprising at least one further process chamber having at least one inlet for water, at least one inlet for at least carbon and at least one outlet, wherein the inlet for at least carbon is directly connected to the at least one outlet of the hydrocarbon converter;
wherein at least one separation unit is provided for separating the carbon obtained by decomposing and the hydrogen obtained by decomposing, the separation unit having separate outlets for the separated materials coming from the separation unit, wherein the outlet for carbon is connected to the C converter, wherein a separate inlet pipe for the hydrogen coming from the separation unit is provided, the separate inlet pipe leading into a mixing chamber located downstream.

11. The apparatus for generating synthesis gas according to claim 9 or 10, wherein the at least one unit for introducing energy into the process chamber is designed in such a way that it can generate, at least locally, temperatures above 1000° C; and/or
wherein the at least one unit for introducing energy into the process chamber comprises a plasma unit in a Kvaerner reactor.

12. The apparatus for generating synthesis gas according to one of the claims 9 to 11, wherein the process chamber of the C converter is formed by an outlet pipe of the hydrocarbon converter, wherein the outlet pipe is connected to an inlet for water.

13. The apparatus for generating synthesis gas according to claim 11, wherein the C converter is further adapted to produce an aerosol comprising carbon and hydrogen.

14. The apparatus for generating synthesis gas according to one of claims 9 to 13 having at least one additional hydrocarbon converter for decomposing a hydrocarbon containing fluid into carbon and hydrogen, the additional hydrocarbon converter comprising:
at least one process chamber having at least one inlet for the hydrocarbon containing fluid; at least one unit for introducing energy into the process chamber, the energy at least partially consisting of heat;
a separation unit for separating the carbon obtained by decomposing and the hydrogen obtained by decomposing, the separation unit having separate outlets for carbon and hydrogen, wherein the outlet for hydrogen is connected to the separate inlet pipe for hydrogen;
wherein the at least one additional hydrocarbon converter is of a type carrying out decomposing at temperatures below 1000°C, particularly below 600°C by means of a microwave plasma.

15. An apparatus for transforming synthesis gas into synthetic functionalised and/or non-functionalised hydrocarbons comprising:
an apparatus according to one of claims 9 to 14; and
a CO converter having a process chamber, in which a catalyst is located, and means for bringing the synthesis gas in contact with the catalyst, and a control unit for the open-loop controlling or closed-loop regulating the temperature of the catalyst and/or the synthesis gas to a predetermined temperature.

16. The apparatus according to claim 15, wherein the CO converter comprises one of the following converters: a Fischer-Tropsch converter, a SMDS converter, a Bergius-Pier converter, a Pier converter, or a combination of a Pier converter with a MtL converter.

## Revendications

1. Procédé de production d'un gaz de synthèse comprenant les étapes suivantes :
décomposer un fluide contenant un hydrocarbure en carbone et en hydrogène par introduction d'énergie, l'énergie étant fournie au moins partiellement par de la chaleur, dans lequel le carbone et l'hydrogène ont une température d'au moins 200°C après l'étape de décomposition ;
amener de l'eau en contact avec au moins une partie du carboneproduit par l'étape de décomposition à une température comprise entre 800 et 1700°C, le carbone obtenu par l'étape de décomposition,après avoir été amené en contact avec l'eau, ayant refroidi de pas plus de 50% en °C par rapport à sa température après l'étape de décomposition ;
transformer au moins une partie de l'eau et du carbone obtenu par l'étape de décompositionen gaz de synthèse ;
dans lequel le carbone obtenu par l'étape de décomposition et l'hydrogène obtenu par l'étape de décompositionsont amenés simultanément en contact avec l'eau.

2. Procédé de production d'un gaz de synthèse comprenant les étapes suivantes :
décomposer un fluide contenant un hydrocarbure en carbone et en hydrogène par introduction d'énergie, l'énergie étant fournie au moins partiellement par de la chaleur, dans lequel le carbone et l'hydrogène ont une température d'au moins 200°C après l'étape de décomposition ;
amener de l'eau en contact avec au moins une partie du carbone produit par l'étape de décomposition à une température comprise entre 800 et 1700°C, le carbone obtenu par l'étape de décomposition,après avoir été amené en contact avec l'eau, ayant refroidi de pas plus de 50% en °C par rapport à sa température après l'étape de décomposition ;
transformer au moins une partie de l'eau et du carbone obtenu par l'étape de décomposition en gaz de synthèse ;
dans lequel le carbone obtenu par l'étape de décomposition est séparé au moins partiellement de l'hydrogène obtenu par l'étape de décomposition avant l'étape de mise du carbone en contact avec l'eau, et
dans lequel au moins une partie de l'hydrogène séparé est ajouté au gaz de synthèse produit par la transformation.

3. Procédé de production d'un gaz de synthèse selon la revendication 1 ou 2, dans lequel l'étape de décomposition prend place à une température supérieure à 1000°C et dans lequel le carbone est amené en contact avec l'eau à une température d'au moins 1000°C, en particulier à une température comprise entre 1000 et 1200°C ; et
dans lequel, de préférence, la chaleur requise pour atteindre la température comprise entre 800 et 1700°C pour la transformation provient pour l'essentiel complètement de la chaleur qui est fournie par la décomposition du fluide contenant un hydrocarbure.

4. Procédé de production d'un gaz de synthèse selon l'une des revendications précédentes,dans lequel au moins une partie de la chaleur d'au moins une partie du carbone obtenu par l'étape de décomposition,et/ou au moins une partie de l'hydrogène obtenu par l'étape de décomposition,et/ou au moins une partie du gaz de synthèse est utilisée pour chauffer l'eau avant d'amener l'eau en contact avec le carbone et/ou est utilisée pour chauffer la chambre de traitement, dans lequel l'eau est amenée en contact avec la carbone et/ou est utilisée pour produire de l'électricité, dans lequel l'électricité peut en particulier être fournie en tant que support d'énergie pour introduire de l'énergie pour décomposer le fluidecontenant un hydrocarbure.

5. Procédé de production d'un gaz de synthèse selon l'une des revendications précédentes, dans lequel l'énergie de décomposition du fluide contenantun hydrocarbure est essentiellement introduite au moyen d'un plasma, et dans lequel l'étape de décomposition est de préférence réalisée dans un réacteur de Kvaerner.

6. Procédé de production d'un gaz de synthèse selon la revendication 5, dans lequel le carbone produit par décomposition et l'hydrogène produit par décomposition sont mis en contact avec l'eau sous forme d'aérosol.

7. Procédé de production d'hydrocarbures de synthèse fonctionnalisés et/ou non fonctionnalisés, dans lequel d'abord un gaz de synthèse est produit selon l'une quelconque des revendications 1 à 8, et dans lequel le gaz de synthèse est amené en contact avec un catalyseur approprié pour provoquer la transformation du gaz de synthèse en deshydrocarbures de synthèse fonctionnalisés et/ou non fonctionnalisés, dans lequel la température du catalyseur et/ou du gaz de synthèse est contrôlée en boucle ouverte ou régulée en boucle fermée dans une plage de températures prédéterminée.

8. Procédé de production d'hydrocarbures de synthèse fonctionnalisés et/ou non fonctionnalisés selon la revendication 7, dans lequel la transformation du gaz de synthèse est effectuée par l'un des processus suivants :un processus de Fischer-Tropsch, un processus SMDS, un processus de Bergius-Pier, un processus de Pier, ou une combinaison d'un processus, de Pier et d'un processus MtL.

9. Dispositif de productiond'un gaz de synthèse comprenant :
un convertisseur d'hydrocarbure pour décomposer un fluide contenant un hydrocarbure en carbone et en hydrogène, le convertisseur d'hydrocarbure comprenant au moins une chambre de traitement ayant au moins une entrée pour un fluide contenant un hydrocarbure et au moins une sortie pour du carbone et/oude l'hydrogène et au moins un module pour introduire de l'énergie dans la chambre de traitement, énergie consistant au moins partiellement en chaleur ;
un convertisseur de C pour la transformation d'eau et de carbone, le convertisseur de C comprenant au moins une autre chambre de traitement ayant au moins une entrée pour l'eau, au moins une entrée pour au moins du carbone et au moins une sortie, l'entrée pour au moins du carbone étant directement reliée à ladite au moins une sortie du convertisseur d'hydrocarbure ;
dans lequel le convertisseur d'hydrocarbure, ladite au moins une sortie du convertisseur d'hydrocarbure, le convertisseur de C et ladite au moins une entrée du convertisseur deC sont adaptés à diriger simultanément le carbone et l'hydrogène produits par le convertisseur d'hydrocarbure dans le convertisseur de C.

10. Dispositif pour produire un gaz de synthèse comprenant :
un convertisseur d'hydrocarbure pour décomposer un fluide contenant un hydrocarbure en carbone et en hydrogène, le convertisseur d'hydrocarbure comprenant au moins une chambre de traitement ayant au moins une entrée pour un fluide contenant un hydrocarbure et au moins une sortie pour du carbone et/ou de l'hydrogène et au moins un module pour introduire de l'énergie dans la chambre de traitement, énergie consistant au moins partiellement en chaleur ;
un convertisseur de C pour la transformation d'eau et de carbone, le convertisseur de C comprenant au moins une autre chambre de traitement ayant au moins une entrée pour l'eau, au moins une entrée pour au moins du carbone et au moins une sortie, l'entrée pour au moins du carbone étant directement reliée à ladite au moins une sortie du convertisseur d'hydrocarbure ;
dans lequel au moins un module de séparation est prévu pour séparer le carbone obtenu par décomposition et l'hydrogène obtenu par décomposition, le module de séparation ayant des sorties séparées pour les matériaux séparés provenant du module de séparation, dans lequel la sortie de carbone est reliée au convertisseur de C, un tube d'entrée séparé pour l'hydrogène provenant du module de séparation étant prévu, le tube d'entrée séparé conduisant dans une chambre de mélange disposée en aval.

11. Dispositif de productiond'un gaz de synthèse selon la revendication 9 ou 10, dans lequel ledit au moins un module pour produire de l'énergie dansla chambre de traitement est conçu de sorte qu'il peut produire, au moins localement, une température supérieure à 1000°C, et/ou dans lequel ledit au moins un module pour introduire de l'énergie dans la chambre de traitement comprend un module plasma dans un réacteur de Kvaerner.

12. Dispositif de productiond'un gaz de synthèse selon l'une des revendications 9 à 11, dans lequel la chambre de traitement du convertisseur de Cest formée par un tube de sortie du convertisseur d'hydrocarbure, le tube de sortie étant relié à une entrée d'eau.

13. Dispositif de productiond'un gaz de synthèse selon la revendication 11, dans lequel le convertisseur de C est en outre adapté à produire un aérosol comprenant du carbone et de l'hydrogène.

14. Dispositif de productiond'un gaz de synthèse selon l'une quelconque des revendications 9 à 13, comprenant au moins un convertisseur d'hydrocarbure supplémentaire pour décomposer un fluide contenant un hydrocarbure en carbone et hydrogène, le convertisseur d'hydrocarbure supplémentaire comprenant :
au moins une chambre de traitement ayant au moins une entrée pour le fluide contenant l'hydrocarbure ;
au moins un module pour introduire de l'énergie dans le chambre de traitement, l'énergie consistant au moins partiellement en chaleur ;
un module de séparation pour séparer le carbone obtenu par la décomposition et l'hydrogène obtenu par la décomposition, le module de séparation ayant des sorties séparées pour le carbone et l'hydrogène, la sortie d'hydrogène étant connectée au tube d'entrée séparé pour l'hydrogène ;
dans lequel ledit au moins un convertisseur d'hydrocarbure supplémentaire est du type effectuant unedécomposition à des températures inférieures à 1000°C, en particulier inférieures à 600°C au moyen d'un plasma microonde.

15. Dispositif de transformationd'un gaz de synthèse en hydrocarbures de synthèse fonctionnalisés et/ou non fonctionnalisés, comprenant :
un disposition selon l'une quelconque des revendications 9 à 14 ; et
un convertisseur de CO comportant une chambre de traitement, dans laquelle un catalyseur est disposé, et des moyens pour amener le gaz de synthèse en contact avec le catalyseur et un module de commande pour la commande en boucle ouverte ou la régulation en boucle fermée de la température du catalyseur et/ou du gaz de synthèse à une température prédéterminée.

16. Dispositif selon la revendication 15, dans lequel le convertisseur de CO comprend l'un des convertisseurs suivants : un convertisseurde Fischer-Tropsch, un convertisseur SMDS, un convertisseur de Bergius-Pier, un convertisseur de Pier, ou une combinaison d'un convertisseur de Pier et d'un convertisseur MtL.
